# EUROPEAN PATENT APPLICATION

(11) **EP 4 509 104 A1**
(43) Date of publication of application: **19.02.2025**
(21) Application number: 23871518.9
(22) Date of filing: 10.08.2023
(51) Int. Cl.: A61F 13/15, A61F 13/53, A61F 13/533

(54) **ABSORBENT ARTICLE AND METHOD FOR MANUFACTURING ABSORBING ELEMENT USED THEREFOR**

(30) Priority: 28.09.2022 JP 2022155166
(71) Applicant: Daio Paper Corporation, Ehime 799-0492 (JP)
(72) Inventor: MORITANI, Akie, Shikokuchuo-shi, Ehime 799-0113 (JP)
(74) Representative: Williams Powell
(86) International application number: PCT/JP2023/029273
(87) International publication number: WO 2024/070276

(57) **Abstract**

An absorbent article having an absorber body of improved shape-retainability, while breakage of packing sheet is reduced, includes an absorbent element having an absorber body formed by mixing and accumulating pulp fibers and high-absorbent particles, and packing sheet of crepe paper packing the body. The element has highly-compressed areas arranged at intervals, each formed by compressing the element in the thickness direction from its top face into the body to form a dent. Areas other than the highly-compressed areas are non-highly-compressed areas with larger thickness and lower density. Each highly-compressed area has no inflection point or folding point. The maximum inscribed circle diameter of any highly-compressed area is 2 to 5 mm, and the perimeter of any highly-compressed area is 1 to 2.5 times the maximum inscribed circle perimeter of the highly-compressed area. The maximum inscribed circle diameter of any non-highly-compressed area is 6.5 mm or less.

## Description

### FIELD OF ART

The present invention relates to an absorbent article, such as a disposable diaper, and a method for producing an absorbent element for use in the absorbent article.

### BACKGROUND ART

Absorbent articles in general have an absorber body therein, which has been formed by mixing and accumulating pulp fibers and high-absorbent polymer particles. Such an absorber body, for improved shape-retainability during and after its manufacture, is usually wrapped in a packing sheet of crepe paper or the like, to form an absorbent element, which is contained in an absorbent article (see, e.g., Patent Publication 1).

The absorbent element of an absorbent article, which is held between the legs of a wearer and subjected to forces in various directions from its sides opposed in the width direction with the motion of the legs in walking or the like, is required to fit well in the crotch section, while it is also required to have shape-retainability for keeping the absorber body from being deformed by distortion, cleavage, or the like. The absorbent element of a diaper is, naturally, required to have ensured absorption performance in the crotch section.

A technique known to improve shape-retainability of an absorber body is to provide highly-compressed areas in a lattice pattern or the like, each formed by compressing an absorbent element in the thickness direction through embossing or the like (see, e.g., Patent Publications 1 and 2). However, in forming the highly-compressed areas, with the packing sheet for packing the absorber body formed of crepe paper, the crepe paper may break at the edges of the highly-compressed areas or between adjacent highly-compressed areas. Such breakage of the packing sheet is not preferable as the high-absorbent polymer particles may escape through the breakage out to the surface of the absorbent article.

### PRIOR ART PUBLICATION

### PATENT PUBLICATION

Patent Publication 1: JP 5709584 B

### SUMMARY OF THE INVENTION

### PROBLEM TO BE SOVED BY THE INVENTION

It is therefore an object of the present invention to improve shape-retainability of the absorber body while breakage of the packing sheet is reduced.

### MEANS FOR SOLVING THE PROBLEM

The present inventor, while doing researches for formation of highly-compressed areas compressed in the thickness direction in an absorber body produced by mixing and accumulating pulp fibers and high-absorbent polymer particles, happened to notice a possibility for improvement in shape-retainability of an absorber body as well as for reduction of breakage in a packing sheet. The absorbent article to be discussed below is based on this finding.

### <First Aspect>

An absorbent article having a crotch section, the absorbent article including:
an absorbent element including an absorber body arranged in a region in a front-back direction including the crotch section, and a packing sheet of crepe paper packing the absorber body,
wherein the absorber body has been formed by mixing and accumulating pulp fibers and high-absorbent particles,
wherein the absorbent element has highly-compressed areas arranged at intervals, each having been formed by compressing the absorbent element in a thickness direction from its top face or under face into the absorber body to form a dent,
wherein, in a region where the highly-compressed areas are arranged, areas other than the highly-compressed areas are non-highly-compressed areas with a larger thickness and a lower density compared to those of the highly-compressed areas,
wherein each of the highly-compressed area has a contour without any inflection point or folding point, a diameter of a maximum inscribed circle of a contour of any of the highly-compressed areas is 2 to 5 mm, and a perimeter of a contour of any of the highly-compressed areas is 1 to 2.5 times a perimeter of the maximum inscribed circle of the contour of the highly-compressed area, and
wherein a diameter of a maximum inscribed circle of a contour of any of the non-highly-compressed areas is 6.5 mm or less.

### <Function and Effect>

The absorbent article according to the present aspect is characterized in arranging highly-compressed areas of particular dimensions and shapes more densely than in the conventional, in an absorbent element formed by preparing an absorber body through mixing and accumulating pulp fibers and high-absorbent polymer particles, and packing the absorber body with crepe paper. That is, the highly-compressed areas are areas of a higher density formed by compression of the absorbent element in the thickness direction through pressurizing (direct pressurizing) into a generally uniform thickness, and constitute the bottoms of dents recessed from the top face or under face of the absorbent element. On the other hand, the non-highly-compressed areas are areas with a larger thickness and a lower density compared to those of the highly-compressed areas, but even in the non-highly-compressed areas, the absorber body is deformed by deformation in the highly-compressed areas dragging the non-highly-compressed areas in the vicinity of the highly-compressed areas to deform, so that the thickness is smaller and the density is higher of a non-highly-compressed area at a location closer to the highly-compressed areas. Accordingly, arrangement of the highly-compressed areas of a certain size more densely than in the conventional absorbent element leads to enlargement of the area wherein the thickness of the non-highly-compressed areas is reducing. This causes less strain (applied tensile force is lower) in the packing sheet during formation of the highly-compressed areas therein, resulting in the packing sheet hardly breakable.

Further, even in the non-highly-compressed areas, the absorber body may advantageously be made thinner compared to the thickness without the highly-compressed areas, as a result of deformation in the highly-compressed areas dragging the non-highly-compressed areas in the vicinity of the highly-compressed areas to deform.

In view of the above, it is important that each highly-compressed area is shaped to contain a sufficiently large circle. It is also important that each highly-compressed area is not excessively long in one direction, or does not have a contour excessively complicated. It is further important that the diameter of the maximum inscribed circle of the contour of any of the non-highly-compressed areas is small so that the highly-compressed areas are not sparse (i.e., so that the non-highly-compressed areas are not elongated in every direction).

As it was conventionally assumed that densely arranging the highly-compressed areas was not preferred, exhibition of such effects with such a simple structure as discussed above was unexpected.

### <Second Aspect>

The absorbent article according to the first aspect, wherein a basis weight of the pulp fibers in the absorber body is 100 to 500 g/m²,
wherein a ratio by weight of the pulp fibers to the high-absorbent polymer particles in the absorber body is 45 : 55 to 65 : 35,
wherein the packing sheet is crepe paper having a stretch at break in a front-back direction of 20 to 35% and a stretch at break in the width direction of 4 to 8%, as determined in accordance with JIS P 8113: 2006,
wherein a thickness of the non-highly-compressed areas are 5 to 13 mm, and
wherein a thickness of the highly-compressed areas is 30 to 50% of the thickness of the non-highly-compressed areas.

### <Function and Effect>

Recently, the proportion of high-absorbent polymer particles is often increased for the purpose of thinning the absorber body in size or the like. However, the increased proportion of high-absorbent polymer particles may cause not only deterioration in shape-retaining property of the highly-compressed areas, but also difficulties in deformation of the absorber body in the non-highly-compressed areas in the vicinity of the highly-compressed areas by deformation in the highly-compressed areas dragging the non-highly-compressed areas in the vicinity of the highly-compressed areas to deform. Further, the high-absorbent polymer particles swollen by absorbing excrete liquid may tend to adhere to each other firmly to disturb diffusion of the excrete liquid (gel blocking), which may lead to difficulties in improving diffusibility. Thus, it is preferred to form the highly-compressed areas by compression within the ranges of the present aspect, with the ratio of the pulp fibers to the high-absorbent polymer particles in the absorber body within the range of the present aspect.

### <Third Aspect>

The absorbent article according to the first or second aspect,
wherein the highly-compressed areas are composed only of highly-compressed areas formed by compressing the absorbent element in a thickness direction from its top face into the absorber body to form a dent.

### <Function and Effect>

According to the absorbent element of the present aspect, both back flow protection and diffusibility are improved. That is, as discussed above, the highly-compressed areas are areas of a higher density formed by compression of the absorbent element in the thickness direction through pressurizing (direct pressurizing) into a generally uniform thickness, and constitute the bottoms of dents recessed from the top face of the absorbent element. On the other hand, the non-highly-compressed areas are areas with a larger thickness and a lower density compared to those of the highly-compressed areas, but even in the non-highly-compressed areas, the absorber body is deformed by deformation in the highly-compressed areas dragging the non-highly-compressed areas in the vicinity of the highly-compressed areas to deform, so that the density of a non-highly-compressed area is higher at a location closer to the highly-compressed areas. Accordingly, capillary action is significant in the highly-compressed areas, which results not only in higher liquid retainability compared to that in the vicinity thereof, but also in liquid being sucked toward the highly-compressed areas. In this way, arranging the highly-compressed areas more densely than in the conventional absorbent element realizes retention of more excrete liquid more securely and further from the skin, hardly resulting in back flow.

The denser arrangement of the highly-compressed areas than in the conventional absorbent element also causes the excrete liquid absorbed in the absorber body to be acted on by the suction through the capillary action in the large number of neighboring highly-compressed areas, to diffuse over a wider range.

### <Fourth Aspect>

The absorbent article according to any one of the first to third aspects,
wherein, in a region where the highly-compressed areas are arranged, where a virtual lattice is defined in which first virtual lines, each extending in a first direction, are arranged at first intervals in a second direction which is inclined at 80 to 90° clockwise with respect to the first virtual lines in plan view, and second virtual lines, each extending in the second direction, are arranged at second intervals in the first direction, and
minimum virtual rectangles are defined by intersections of the first virtual lines and the second virtual lines as vertices,
the highly-compressed areas are composed of first highly-compressed areas, each located at an intersection of a first virtual line and a second virtual line, second highly-compressed areas, each located between adjacent first highly-compressed areas on a first virtual line or between adjacent first highly-compressed areas on a second virtual line, and third highly-compressed areas, each located at an intersection of diagonals of a virtual rectangle,
wherein each of the first highly-compressed areas is in a form of a circle having its center at an intersection of a first virtual line and a second virtual line,
wherein each of the second highly-compressed areas has its center of gravity at a midpoint of a side of a virtual rectangle, and is in a form of an ellipse or a rounded rectangle having its long axis extending along the side, and
wherein each of the third highly-compressed areas has its center of gravity at the intersection of the diagonals of a virtual rectangle, and is in a form of an ellipse or a rounded rectangle having its long axis extending in the width direction, or a circle having its center at the intersection of the diagonals of a virtual rectangle.

### <Function and Effect>

In the region where the highly-compressed areas are arranged, the shapes and arrangement of the highly-compressed areas may suitably be decided. With a pattern according to the present aspect, diffusibility in the direction of the long axis of the second highly-compressed areas (the first and second directions) may be improved, and non-highly-compressed areas linearly continued in the first direction and non-highly-compressed areas linearly continued in the second direction are both provided, so that the absorber body as a whole is easily deformable to the body surface, even if each highly-compressed area is hardened.

### <Fifth Aspect>

The absorbent article according to the fourth aspect,
wherein the first highly-compressed areas are 1 to 4 mm in diameter,
wherein the second highly-compressed areas are 3 to 6 mm in long axis, and are of same length in short axis as the diameter of the first highly-compressed areas,
wherein the third highly-compressed areas have same dimensions and same shape as those of the second highly-compressed areas, except that each third highly-compressed area has a long axis aligned to the width direction, and
wherein a shortest distance between adjacent first and second highly-compressed areas is 1 to 2 mm.

### <Function and Effect>

When the highly-compressed areas are to be arranged along the above-discussed virtual lattice, the dimensions of each highly-compressed area may suitably be decided and, within the ranges of the present aspect, not only the protection against packing sheet breakage, the back flow protection, and the liquid diffusibility, but also deformability of the absorbent body is improved, which is preferred.

### <Sixth Aspect>

The absorbent article according to the fourth or fifth aspect,
wherein the virtual lattice is a rhombic lattice in which the first virtual lines are inclined at 40 to 50° clockwise with respect to the front-back direction in plan view, while the second virtual lines are inclined at 40 to 50° counterclockwise with respect to the front-back direction in plan view.

### <Function and Effect>

When the highly-compressed areas are to be arranged along the above-discussed virtual lattice, and along the rhombic lattice according to the present aspect, not only the deformability of the absorber body upon wearing the article, but also liquid diffusibility is superior, which is preferred.

### <Seventh Aspect>

A method for producing an absorbent element having an absorber body formed by mixing and accumulating pulp fibers and high-absorbent polymer particles, and a packing sheet of crepe paper packing the absorber body, the method including:
a first step of preparing an absorber body by mixing and accumulating the pulp fibers and the high-absorbent polymer particles,
a second step of preparing a packed body by packing an entirety of the absorber body with the packing sheet, and
a third step of holding the packed body between an anvil roll having on its roll surface protrusions arranged at intervals and a plain roll having a cylindrical face facing the anvil roll, and compressing only areas of the packed body caught between the number of protrusions on the anvil roll and the plain roll,
wherein, when an arrangement of tip faces of the protrusions of the anvil roll developed in a plan view, in a region where the tip faces are arranged, a diameter of a maximum inscribed circle of a contour of the tip face of any of the protrusions is 2 to 5 mm, a perimeter of a contour of the tip face of any of the protrusions is 1 to 2.5 times a perimeter of the maximum inscribed circle of the contour of the tip face of the protrusion, and a diameter of a maximum inscribed circle of a contour of any area in the region, where the tip faces are arranged, other than the tip faces is 6.5 mm or less.

### <Function and Effect>

This aspect provides the same function and effect as the first aspect.

### EFFECT OF THE INVENTION

According to the present invention, advantages are provided, such as improvement in shape-retainability of the absorber body while breakage of the packing sheet is reduced.

### BRIEF DESCRIPTION OF THE DRAWINGS

Fig. 1 is a plan view of an underpants-type disposable diaper in its spread state, showing its internal face.
Fig. 2 is a plan view of the underpants-type disposable diaper in its spread state, showing its external face.
Fig. 3 is a cross-sectional view taken along lines 2-2 in Fig. 1.
Fig. 4 is a cross-sectional view taken along lines 3-3 in Fig. 1.
Fig. 5(a) is a sectional view taken along lines 4-4 in Fig. 1, and Fig. 5(b) is a sectional view taken along lines 5-5 in Fig. 1.
Fig. 6 is a perspective view of the underpants-type disposable diaper.
Fig. 7 is a plan view of the inner member in its spread state, showing its external face together with the outline of the outer members.
Fig. 8 is a plan view of the absorber body, showing its top face together with the outline of the packing sheet.
Fig. 9 is a sectional view of the absorbent element.
Fig. 10 is a plan view of the absorbent element.
Fig. 11 is an enlarged plan view of a relevant part of the top face of the absorbent element.
Fig. 12 illustrates plan views of other embodiments of the absorber body.
Fig. 13 is a sectional view of the absorbent element before the absorber body is packed in the packing sheet.
Fig. 14 is a sectional view of the absorbent element formed by packing the absorber body with the packing sheet, before the highly-compressed areas are formed therein.
Fig. 15 is a sectional view of another absorbent element.
Fig. 16 is an enlarged plan view of a relevant part of the top face of an absorbent element.
Fig. 17 is an enlarged plan view of a relevant part of the top face of an absorbent element.
Fig. 18 is an enlarged plan view of a relevant part of the top face of an absorbent element.
Fig. 19 is an enlarged plan view of a relevant part of the top face of an absorbent element.

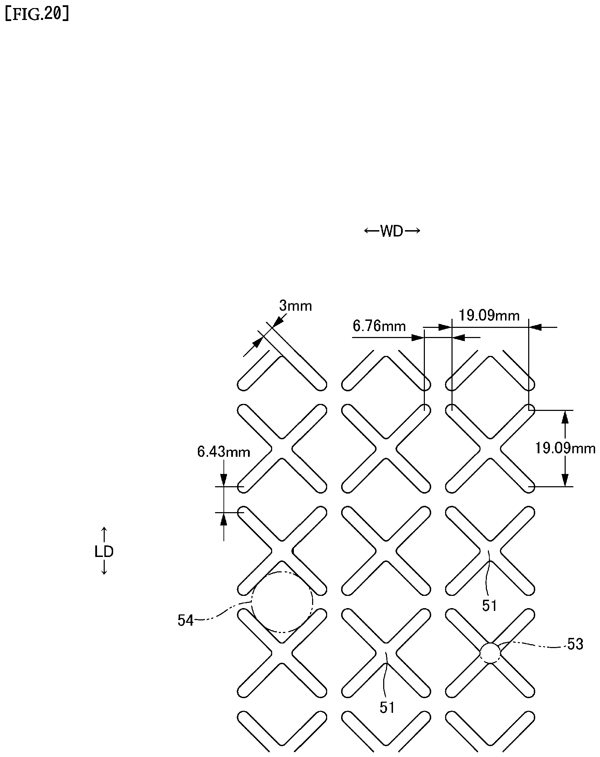

Fig. 20 is an enlarged plan view of a relevant part of the top face of an absorbent element.

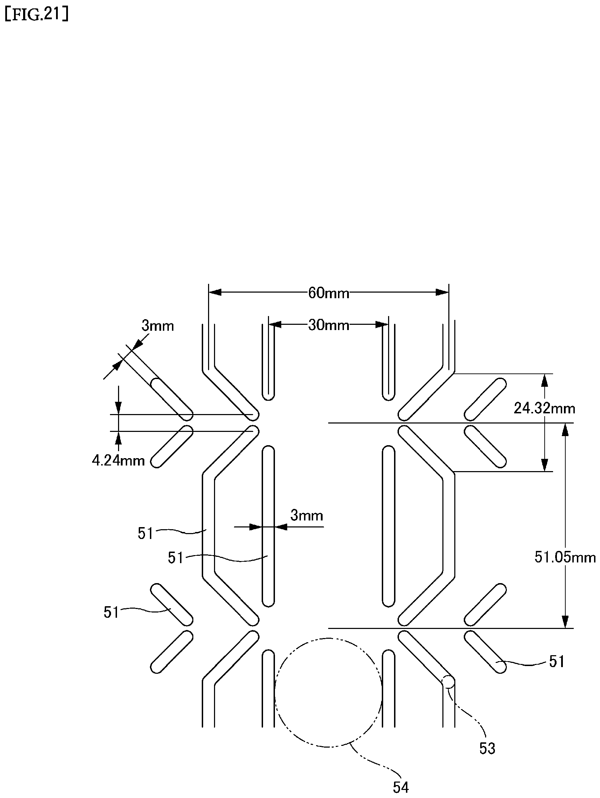

Fig. 21 is an enlarged plan view of a relevant part of the top face of an absorbent element.
Fig. 22 is a schematic view illustrating a method for producing an absorbent element.
Fig. 23 is an explanatory view for illustrating a method for measuring the depth of a highly-compressed area.

### MODES FOR CARRYING OUT THE INVENTION

An underpants-type disposable diaper, as an examples of the absorbent article, will now be discussed in detail with reference to the attached drawings. Note that components adjacent to each other in the thickness direction may be fixed or joined as necessary in the same way as in known diapers, at locations other than the fixed or joined areas to be discussed below. Dotted pattern regions in the sectional views represent an adhesive, such as a hot melt adhesive, as means of the fixing or joining. The hot melt adhesive may be applied by any known manner, such as slot coating, continuous or intermittent linear bead coating, spray coating in spiral, Z-shaped, wave, or the like patterns, or pattern coating (transfer of a hot melt adhesive by relief printing). In place of or in addition to this, on fixing portions of elastic members, a hot melt adhesive may be applied to the external surface of the elastic members for fixing to adjacent members. Examples of the hot melt adhesive include, but not limited to, EVA-based, adherent rubber-based (elastomer-based), polyolefin-based, and polyester/polyamide-based adhesives. The fixing or joining means for joining components may alternatively be material melt-bonding, such as heat sealing or ultrasonic sealing. Components adjacent to each other in the thickness direction are fixed or joined in an intermittent pattern, where liquid permeability in the thickness direction is required. For example, for such intermittent fixing or joining with a hot melt adhesive, intermittent pattern coating in a spiral, Z, wave, or the like shape may preferably be employed. For application in an area wider than the application width of one nozzle, intermittent pattern coating in a spiral, Z, wave, or the like shape may be employed with or without space in the width direction. The joining means for joining components may be material melt-bonding, such as heat sealing or ultrasonic sealing.

As the nonwoven fabric in the description hereinbelow, commonly known nonwoven fabric may suitably be used depending on the parts or purposes. Examples of the constituent fibers of the nonwoven fabric include, but not limited to, synthetic fibers, such as polyolefin-based, e.g., polyethylene or polypropylene, polyester-based, or polyamide-based fibers (including not only single component fibers, but also composite fibers, such as of core/sheath type), as well as regenerated fibers, such as rayon or cupra, or natural fibers, such as cotton, and also mixtures thereof. For improved flexibility of the nonwoven fabric, the constituent fibers may preferably be crimped fibers. The constituent fibers of the nonwoven fabric may also be hydrophilic fibers (including those rendered hydrophilic with hydrophilizers), hydrophobic fibers, or water-repelling fibers (including those rendered water-repelling with water repellents). Further, nonwoven fabric may generally be categorized into discontinuous fiber nonwoven, continuous fiber nonwoven, spunbonded nonwoven, melt blown nonwoven, spunlace nonwoven, thermal bonded (air through) nonwoven, needle-punched nonwoven, point-bonded nonwoven, composite nonwoven (including SMS or SMMS nonwoven fabric having one or more melt blown layers interposed between spunbonded layers), or the like nonwoven fabric, generally depending on the length of the fibers, method of forming the sheet, method of joining the fibers, or layered structure, and any of these nonwoven fabric may be used.

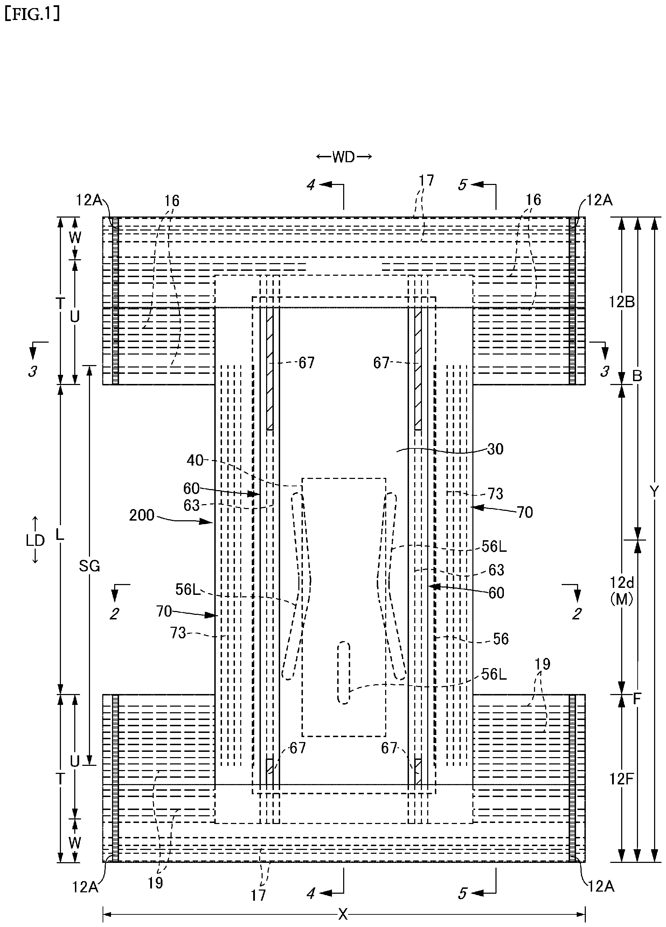

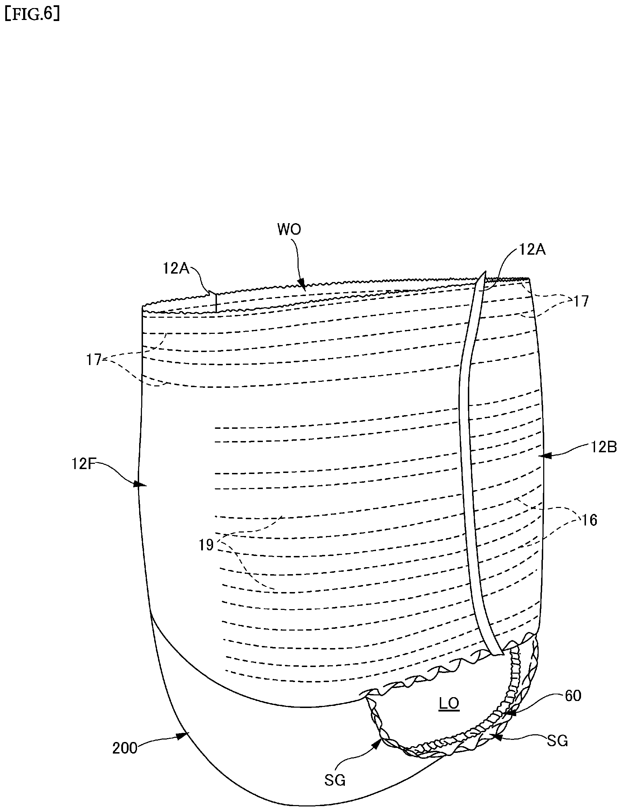

Figs. 1 to 6 show an embodiment of an underpants-type disposable diaper. The underpants-type disposable diaper according to the invention has a rectangular front outer member 12F forming a front lower torso section, a rectangular back outer member 12B forming a back lower torso section, and an inner member 200 provided on the inner faces of the outer members 12F, 12B to extend from the front outer member 12F, via crotch section M, to the back outer member 12B. The front outer member 12F and the back outer member 12B are joined along their opposed lateral sides to form a pair of side seals 12A, to thereby define an opening with the front and back edges of the outer members 12F, 12B, which is a waist opening WO for passing the torso of the wearer therethrough, whereas a pair of leg openings LO is formed on either side in the width direction WD of the inner member 200, defined by the lower edges of the outer members 12F, 12B and the side edges of the inner member 200. The inner member 200 absorbs and holds bodily waste, such as urea, while the outer members 12F, 12B support the inner member 200 with respect to the body of a wearer. Reference sign Y refers to the entire length of the diaper in its spread state (the length in the front-back direction from the edge of the waist opening WO in the front body section F over to the edge of the waist opening WO in the back body section B), whereas reference sign X refers to the entire width of the diaper in its spread state.

The underpants-type disposable diaper according to the present embodiment has lower torso regions T defined as extents in the front-back direction over the side seals 12A (zones each extending in the front-back direction from the waist opening WO to the upper edge of a leg opening LO) and an intermediate section L defined as an extent in the front-back direction forming the leg openings LO (a region between the front-back region of the front body section F along the side seals 12A and the front-back region of the back body section B along the side seals 12A). The regions located in the lower torso regions T of the front outer member 12F and the back outer member 12B, i.e., the front lower torso section and the back lower torso section may each be divided conceptually into a waist zone W forming the edge zone of the waist opening and an under-waist zone U extending downward of the waist zone W. Usually, when the front lower torso section and the back lower torso section each has one or more boundaries between zones of different stretching stresses in the width direction WD (e.g., with elastic members of different finenesses or stretch rates), the zone on the waist opening WO side of the boundary closest to the waist opening WO is the waist zone W. In the absence of such boundaries, the zones of the front and back lower torso sections extending toward the waist opening WO beyond the absorber body 56 or the inner member 200 are the waist zones W. The length in the front-back direction of the waist zones may vary depending on the size of the product, and may suitably be decided. For example, each waist zone W may extend over 15 to 40 mm and each under-waist zone U may extend over 65 to 120 mm. On the other hand, the side edges of the intermediate section L are centrally narrowed in square U-shapes or curved shapes to fit the round-leg profile of the wearer, and the legs of the wearer are to be inserted here. Thus, underpants-type disposable diapers in the spread state have generally an hourglass shape as a whole.

### <Outer Member>

The outer members 12F, 12B are composed of a rectangular front outer member 12F forming at least the lower torso section of the front body section F and a rectangular back outer member 12B forming at least the lower torso section of the back body section B, as in the illustrated embodiment, and the front outer member 12F and the back outer member 12B may be non-continuous on their crotch sides and spaced apart in the front-back direction LD (two-segmented outer member). Alternatively, though not shown, the front body section F and the back body section B may be continuous (integral outer member). The spaced distance 12d in the front-back direction in the two-segmented outer member may be, for example, about 40 to 60% the overall length Y. In the illustrated embodiment, the lower edges of the front outer member 12F and the back outer member 12B extend linearly along the width direction WD, but at least one of the lower edges of the front and back outer members 12F and 12B may be curved along the round-leg profile.

The underpants-type disposable diaper with the two-segmented outer member, wherein the inner member 200 is exposed between the front outer member 12F and the back outer member 12B, is preferably provided, on the under face of the inner member 200, with a covering nonwoven layer 13 extending from an overlap portion between the front outer member 12F and the inner member 200 to an overlap portion between the back outer member 12B and the inner member 200 so that a liquid impervious film 11 is not exposed on the under face of the inner member 200. The inner and outer faces of the covering nonwoven layer 13 may be adhered to the surfaces facing these faces, respectively, via a hot melt adhesive. The covering nonwoven layer 13 may be made of, for example, nonwoven fabric suitably selected from those forming the outer members 12F, 12B. Though not shown, the outer member may be continuous from the front body section F through the crotch section to the back body section B. In this case, the outer member not only has regions corresponding to the lower torso regions T, but also has regions corresponding to the intermediate sections L.

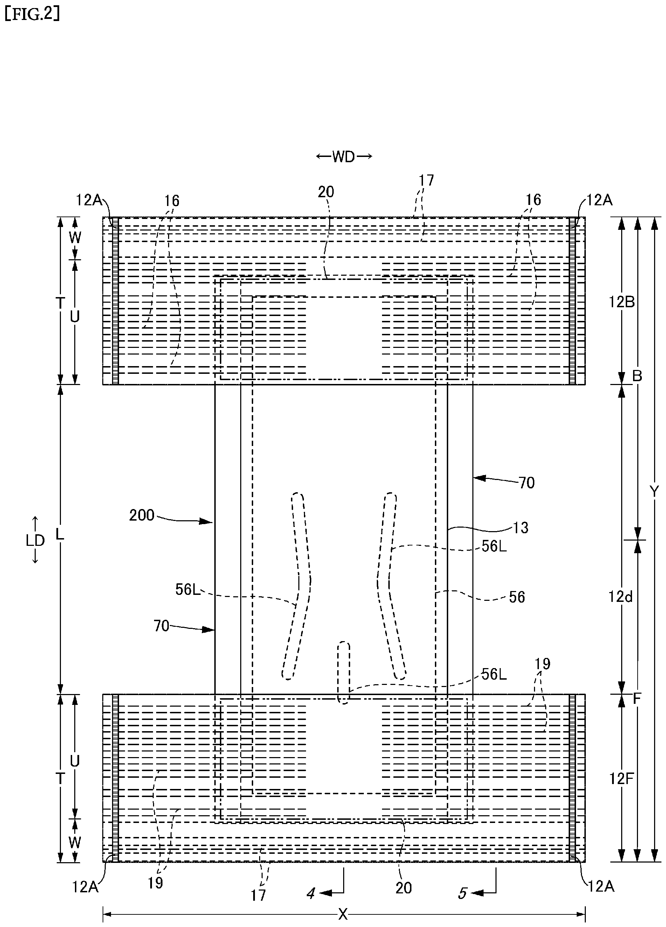

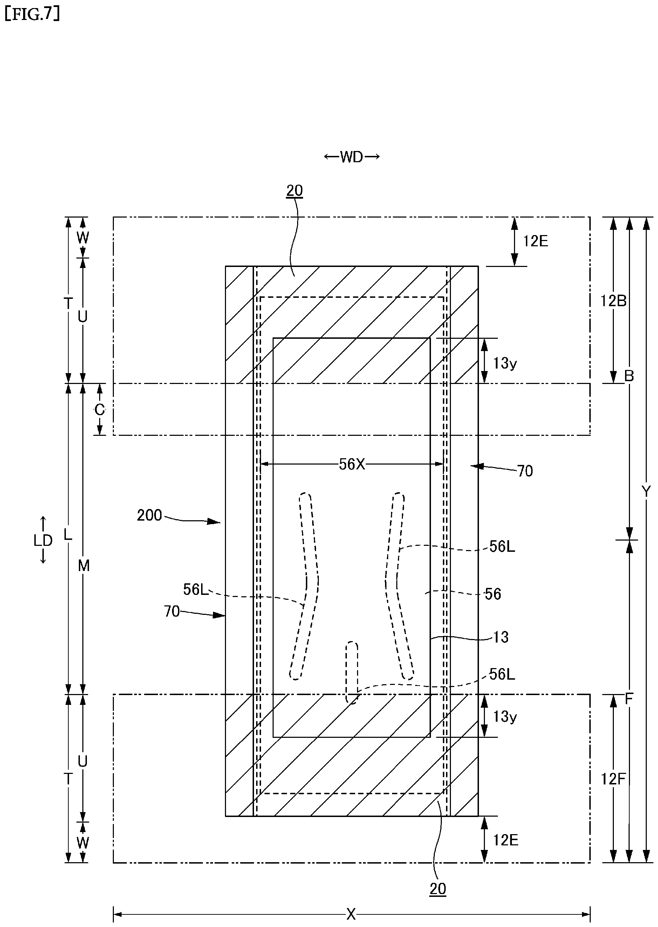

The front outer member 12F and the back outer member 12B have the front lower torso section and the back lower torso section, respectively, forming the lower torso regions T. In the embodiment shown in Figs. 1 and 2, the front outer member 12F and the back outer member 12B have the same dimension in the front-back direction LD, and neither the front outer member 12F nor the back outer member 12B has any zone corresponding to the intermediate region L. However, as shown in Fig. 7, the back outer member 12B may have a larger dimension in the front-back direction compared to the front outer member 12F, the front outer member 12F may have no zone corresponding to the intermediate region L, but the back outer member 12B may have a buttocks-covering zone C extending from the lower torso region T toward the intermediate region L. Though not shown, the front outer member 12F may also have a groin-covering zone extending from the lower torso region T toward the intermediate region L.

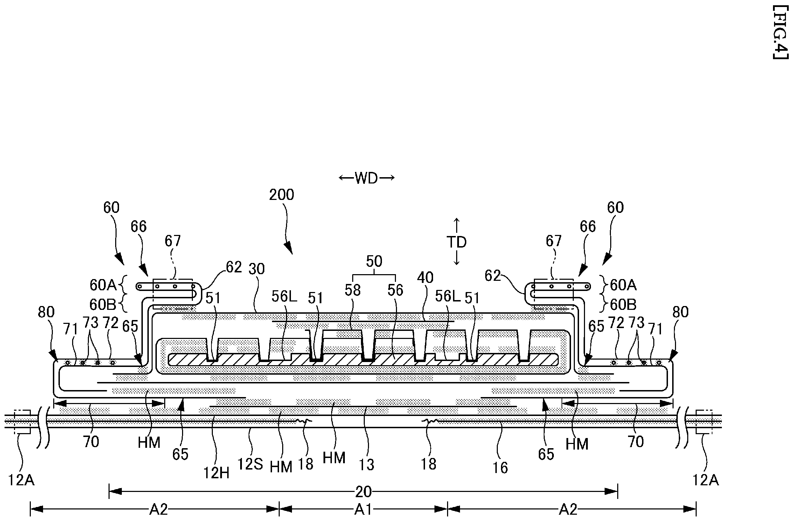

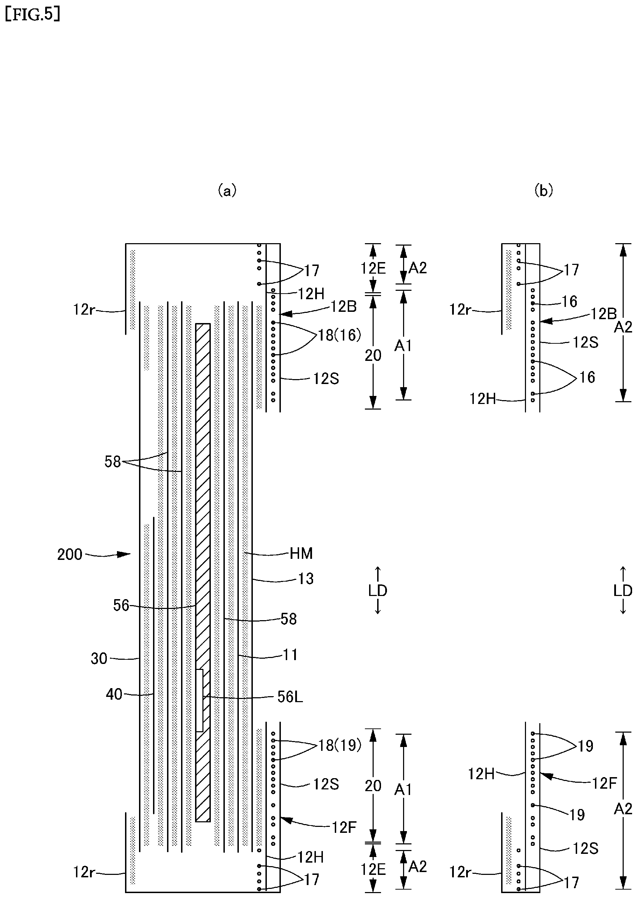

Each outer member 12F, 12B is formed by joining an outer sheet layer and an inner sheet layer adjacent outward and inward, respectively, to elastic members 16 to 19 to be discussed later, with joining means, such as a hot melt adhesive or melt-bonding, as shown in Figs. 4 and 5. The outer sheet layer and the inner sheet layer may be formed of two sheet materials 12S and 12H as in the illustrated embodiment, or may be formed of a single sheet material. For example, in the case of the latter, the inner sheet layer and the outer sheet layer may be formed of the inner section and the outer section, respectively, of a single sheet material folded along the edge of the waist opening WO (or the crotch-side edges) in part of or all over the outer members 12F, 12B. The illustrated embodiment represents the former, wherein the sheet material 12S forming the outer sheet layer in the under-waist zone is folded inward around the waist opening WO side of the sheet material 12H forming the inner sheet layer in the under-waist zone, and the resulting folded part 12r extends to cover the waist opening WO side end of the inner member 200. On the other hand, in the waist zone, the folded part 12r is the inner sheet layer adjacent inward to the elastic members.

For improved fitting on the lower torso of the wearer, each outer member 12F, 12B is provided with elastic members 16 to 19 therein to form a stretchable region A2, which is elastically stretchable with the stretching/contracting of the elastic members 16 to 19 in the width direction WD. In this stretchable region A2, each of the outer members 12F, 12B in the natural length state is contracted with the contraction of the elastic members to form creases or ridges therein, and is stretchable in the longitudinal direction of the elastic members up to the predetermined stretch rate where the stretchable region is fully stretched without creases. The elastic members 16 to 19 may be elongate elastic members, such as rubber threads (as illustrated in the figures), or any conventionally known elastic members, such as of a tape, net, or film shape, without particular limitation. The elastic members 16 to 19 may either be made of synthetic rubber or natural rubber.

The elastic members 16 to 19 in the illustrated embodiment will now be discussed in further detail. In the waist zone W of each outer member 12F, 12B, a plurality of waist elastic members 17 is attached at intervals in the front-back direction, with each member 17 extending continuously all over the width direction WD. One or a plurality of the waist elastic members 17 arranged adjacent to the under-waist zone U may be arranged coincident with the inner member 200, or may be arranged on the opposed lateral sides in the width direction of the inner member 200, except for a middle region in the width direction thereof, which is overlapped with the inner member 200. As the waist elastic members 17, about two to fifteen, particularly about four to ten rubber threads, each having a fineness of 155 to 1880 dtex, particularly about 470 to 1240 dtex (in the case of synthetic rubber) (in the case of natural rubber, with a cross-sectional area of 0.05 to 1.5 mm², particularly about 0.1 to 1.0 mm²), are preferably arranged at 2 to 12 mm intervals, particularly 3 to 7 mm intervals, to render the waist zone W to have a stretch rate in the width direction WD of preferably 150 to 400%, particularly about 220 to 320%. Further, the waist zone W may not necessarily contain the elastic members of the same fineness or the same stretch rate all over the front-back direction LD, and may contain elastic members having partially different finenesses or stretch rates.

In the under-waist zone U of each outer member 12F, 12B, a plurality of under-waist elastic members 16, 19, which is a plurality of elongate elastic members, is attached at intervals in the front-back direction, to form under-waist stretchable regions (regions containing the under-waist elastic members 16, 19). As the under-waist elastic members 16, 19, about five to thirty rubber threads, each having a fineness of 155 to 1880 dtex, particularly about 470 to 1240 dtex (in the case of synthetic rubber) (in the case of natural rubber, with a cross-sectional area of 0.05 to 1.5 mm², particularly about 0.1 to 1.0 mm²), are preferably arranged at 1 to 15 mm intervals, particularly 3 to 8 mm intervals, to render the under-waist zone U to have a stretch rate in the width direction WD of preferably 200 to 350%, particularly about 240 to 300%. Further, the under-waist zone U may not necessarily contain the elastic members of the same fineness or the same stretch rate all over the front-back direction LD, and may contain elastic members having partially different finenesses or stretch rates.

When the elastic members 16, 19 are provided in a zone extending in the front-back direction and coincident with the absorber body 56 as in the under-waist zone U in the illustrated embodiment, in order to avoid contraction in the width direction WD of the absorber body 56 partially or entirely, it is preferred, as shown in Figs. 4, 5, 12, and others, that the middle region in the width direction of the under-waist zone U containing part or all of the part of the elastic members 16, 19 coincident with the absorber body 56 in the width direction WD is made a non-stretchable region A1, and the regions on the opposed lateral sides in the width direction thereof are made stretchable regions A2 (under-waist stretchable regions in the illustrated embodiment). The dimension in the width direction of each stretchable region A2 arranged on the opposed lateral sides in the width direction of the non-stretchable region A1 may be approximately constant in the front-back direction LD as in the illustrated embodiment, or may vary in the front-back direction LD, though not shown. The dimension in the width direction of each stretchable region A2 arranged on the opposed lateral sides in the width direction of the non-stretchable region A1 may either be approximately the same or different between the front body section F and the back body section B.

Such stretchable regions A2 and non-stretchable region A1 may be formed by attaching the elastic members 16 to 17, 19 between the inner sheet layer and the outer sheet layer, and finely dividing the elastic members 16, 19 by applying pressure and heat or by cutting at a single location in the middle in the width direction or almost all over the region to be the non-stretchable region A1, to thereby wreck the stretchability in the non-stretchable region A1, while the stretchability in the stretchable regions A2 is left intact. Naturally, unnecessary elastic members 18 are to be left in the non-stretchable region A1, which do not substantially contribute to the stretchability.

The sheet material for the inner sheet layer 12H and the outer sheet layer 12S may be any material without particular limitation, and may preferably be nonwoven fabric. The nonwoven fabric, where used, preferably has a basis weight of about 10 to 30 g/m² per sheet.

The elastic members 16 to 19 may be fixed to the outer members 12F, 12B by any commonly known manner. Similarly, the inner sheet layer and the outer sheet layer may be joined to each other by any commonly known manner. For example, in the regions of the outer members 12F, 12B containing the elastic members 16 to 19, by applying a hot melt adhesive HM only to the circumferential surfaces of the elastic members 16 to 19 using application means, such as comb guns or surewrap nozzles, and holding the elastic members between the inner sheet layer and the outer sheet layer, not only the fixing of the elastic members 16 to 19 to the inner and outer sheet layers, but also the fixing between the inner and outer sheet layers may be achieved by means of only the hot melt adhesive applied on the circumferential surfaces of the elastic members 16 to 19.

### <Inner Member Joint Area>

The inner member 200 may be fixed to the outer members 12F, 12B by joining means, such as material melt-bonding, including heat sealing or ultrasonic sealing, or with a hot melt adhesive. In the illustrated embodiment, the under face of the inner member 200 is fixed to the internal faces of the outer members 12F and 12B via a hot melt adhesive applied to the under face of liquid-impervious sheet 11 and root portion 65 of standup gather parts 60. This inner member joint area 20 joining the inner member 200 and the outer members 12F, 12B may be provided almost all over the overlapping area between the inner member and each outer member as shown in Fig. 2, for example, in the region except for the regions on the opposed lateral sides in the width direction of the inner member 200.

### <Inner Member>

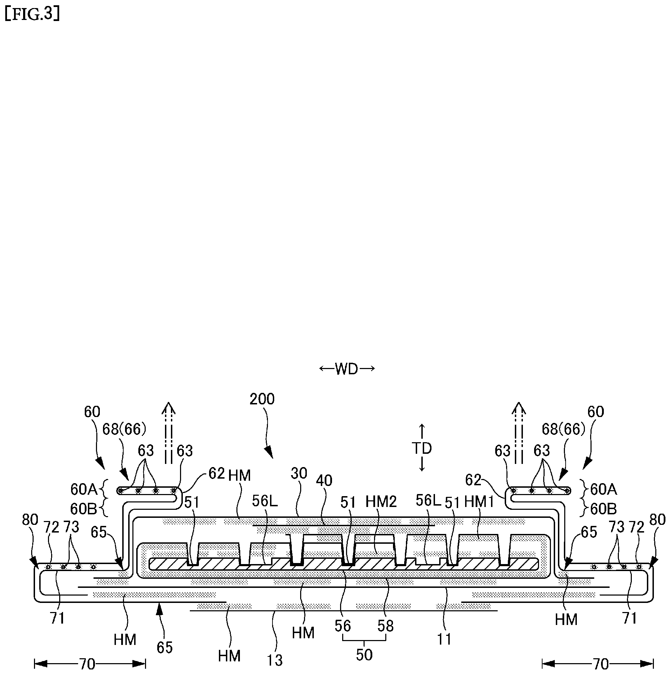

The inner member 200 may be in any shape and, in the illustrated embodiment, is in a rectangular shape having long sides extending in the front-back direction LD. The inner member 200 has a top sheet 30 to be disposed on the side of the skin of the wearer, a liquid impervious sheet 11, and an absorbent element 50 interposed therebetween, as shown in Figs. 3 to 5. Reference numeral 40 refers to an intermediate sheet (second sheet) interposed between the top sheet 30 and the absorbent element 50 so as to have the liquid permeated through the top sheet 30 promptly transferred to the absorbent element 50. Reference numeral 60 refers to a standup gather part extending from each lateral side of the inner member 200 so as to touch around a leg of the wearer in order to keep the bodily waste from leaking through the lateral sides of the inner member 200.

### <Top Sheet>

The top sheet 30 has a property to allow permeation of liquid therethrough, and may be, for example, perforated or non-perforated nonwoven fabric or perforated plastic sheet. Further, the top sheet 30 may be formed of a single sheet or a layered sheet prepared by bonding two or more sheets together. Similarly, the top sheet 30 may be formed, in the planar direction, of a single sheet or two or more sheets.

The top sheet 30 may be folded in each lateral side along the side edge of the absorbent element 50 to extend toward the underside thereof, or may not be folded to extend laterally beyond the side edge of the absorbent element 50.

The top sheet 30 is preferably fixed to a member adjacent underside thereof by means of joining means through material melt-bonding, such as heat sealing or ultrasonic sealing, or a hot melt adhesive, for the purpose of keeping the top sheet 30 from displacing with respect to the member underside thereof. In the illustrated embodiment, the top sheet 30 is fixed to the top face of the intermediate sheet 40 and to the top face of part of the packing sheet 58 that is located on the top side of the absorber body 56, by means of a hot melt adhesive applied to the underside of the top sheet 30.

### <Intermediate Sheet>

An intermediate sheet (also referred to as a second sheet) 40 may be provided, which has a higher liquid transmission rate compared to the top sheet 30, so as to have the liquid permeated through the top sheet 30 promptly transferred to the absorber body 56. This intermediate sheet 40 promptly transfers liquid to the absorber body 56 to improve the absorption performance of the absorber body 56, while it keeps the absorbed liquid from "back flowing" from the absorber body 56. The intermediate sheet 40 may be omitted.

The intermediate sheet 40 may be made of, for example, a material similar to that of the top sheet 30, spunlace nonwoven fabric, spunbonded nonwoven fabric, SMS nonwoven fabric, pulp nonwoven fabric, mixed sheet of pulp and rayon, point-bonded nonwoven fabric, or crepe paper. Air through nonwoven fabric is particularly preferred for its bulkiness. For the air through nonwoven fabric, composite fibers having a core-in-sheath structure are preferably used, in which the resin use for the core may be polypropylene (PP), but may preferably be polyester (PET), which is highly rigid. The basis weight is preferably 17 to 80 g/m², more preferably 25 to 60 g/m². The fineness of the raw material fibers of the nonwoven fabric is preferably 2.0 to 10 dtex. For making the nonwoven fabric bulky, it is preferred to use eccentric fibers having the core off the center, hollow fibers, or eccentric and hollow fibers, as mixed fibers constituting all or part of the raw material fibers.

The intermediate sheet 40 in the illustrated embodiment is shorter than the width of the absorber body 56 and positioned in the center, but may be provided all over the width of the absorber body 56. The dimension of the intermediate sheet 40 in the front-back direction may be the same as the overall length of the diaper, the same as the length of the absorbent element 50, or within a short length range around the area in which liquid is absorbed.

The intermediate sheet 40 is preferably fixed to a member adjacent underside thereof by means of joining means through material melt-bonding, such as heat sealing or ultrasonic sealing, or a hot melt adhesive, for the purpose of keeping the intermediate sheet 40 from displacing with respect to the member underside the intermediate sheet 40. In the illustrated embodiment, the intermediate sheet 40 is fixed to the top face of part of the packing sheet 58 that is located on the top side of the absorber body 56, by means of a hot melt adhesive applied to the underside of the intermediate sheet 40.

### <Liquid-Impervious Sheet>

The liquid-impervious sheet 11 may be made of any material without particular limitation, for example, plastic film made of a polyolefin-based resin, such as polyethylene or polypropylene, laminated nonwoven fabric composed of nonwoven fabric and plastic film provided over the nonwoven fabric, a layered sheet composed of plastic film and nonwoven fabric or the like, layered and joined together. The liquid-impervious sheet 11 is preferably made of a material having both liquid-impermeability and moisture-permeability, which material is preferably used for preventing dampness. Such moisture-permeable plastic film may be microporous plastic film, which is widely used and obtained by kneading an inorganic filler in a polyolefin-based resin, such as polyethylene or polypropylene, forming the resulting mixture into a sheet, and then uni- or biaxially drawing the sheet. Also, nonwoven fabric of microdenier fibers, nonwoven fabric that has been given an enhanced leak proof property by applying heat or pressure to minimize interfiber gaps, or sheets that have been rendered liquid-impervious without using plastic film through a process, such as coating with a highly water-absorbable resin or a hydrophobic resin or water repellent, may be used as the liquid-impervious sheet 11. For sufficient bonding strength in bonding to a cover nonwoven sheet 13 via a hot melt adhesive as will be discussed later, use of resin film is preferred.

The liquid-impervious sheet 11 may have a width that fits behind the absorbent element 50 as illustrated, or may wrap around the edges of the absorbent element 50 to extend to the opposed sides of the top sheet 30 of the absorbent element 50 for improved leak proof property. The width of such extensions may suitably be 5 to 20 mm on each of the right and left sides.

### <Absorbent Element>

The absorbent element 50 includes the absorber body 56 and a packing sheet 58 that entirely wraps the absorber body 56. The absorbent element 50 has a crotch section M and sections each extending forward of or backward of the crotch section M.

### <Absorber Body>

The absorber body 56 is formed by mixing and accumulating pulp fibers and high-absorbent polymer particles, and the pulp fibers and the high-absorbent polymer particles are present generally uniformly throughout the absorber body 56. The pulp fibers may have a basis weight of, for example, about 100 to 450 g/m². The absorber body 56 is preferably formed by mixing and accumulating pulp fibers and high-absorbent polymer particles only, but may contain a minute amount of additives, such as deodorant or perfume, as long as the additives do not disturb improvement in back flow protection and diffusibility as will be discussed later.

The high-absorbent polymer particles include not only "particles", but also "powders". The high-absorbent polymer particles may be those used in this type of disposable diapers as they are, and may preferably be particles 30 wt% or less of which, after sieving (five-minute shaking), remain on a 500 µm standard sieve (JIS Z8801-1: 2006) and particles 60 wt% or more of which, after sieving (five-minute shaking), remain on a 180 µm standard sieve (JIS Z8801-1: 2006).

Any material of the high-absorbent polymer particles may be used without particular limitation, and those having a water absorption of 40 g/g or more are preferred. The high-absorbent polymer particles may be starch-based, cellulose-based, or synthetic polymer-based, and starch-acrylic acid (salt) graft copolymers, saponified products of starch-acrylonitrile copolymers, cross-linked sodium carboxymethyl cellulose, acrylic acid (salt) polymers, or the like, may be used. The high-absorbent polymer particles may preferably be in ordinary powder or granular form, but particles in other forms may also be used.

The high-absorbent polymer particles having a water absorption rate of 70 seconds or less, particularly 40 seconds or less, may preferably be used. With too slow a water absorption rate, so-called back flow may likely to occur, in which liquid supplied into the absorber body 56 returns out of the absorber body 56.

The high-absorbent polymer particles may preferably have a gel strength of 1000 Pa or higher. With such property, when the high-absorbent polymer particles are formed into a bulky absorber body 56, stickiness after liquid absorption may effectively be limited.

The basis weight of the high-absorbent polymer particles may suitably be decided depending on the amount of absorption required in a use of the absorber body 56. Thus, it depends, but the basis weight may be 50 to 350 g/m². At a basis weight of the polymer less than 50 g/m², the amount of absorption may hardly be secured. At over 350 g/m², the effect may be saturated.

The ratio of the fibers to the high-absorbent polymer particles in the absorber body 56 is not particularly limited, and may be 40 : 60 to 65 : 35 by weight.

The thickness 56t of the absorber body 56 is not particularly limited, and may be 5 to 13 mm.

It suffices that the absorber body 56 extends continuously from forward of the crotch section M to backward of the crotch section M to include the crotch section M. In an underpants-type disposable diaper like this embodiment, the absorber body 56 extends in the front-back direction LD and in the width direction WD preferably up to or to the vicinity of the peripheral edges of the inner member 200. Reference sign 56X refers to the maximum width of the absorber body 56.

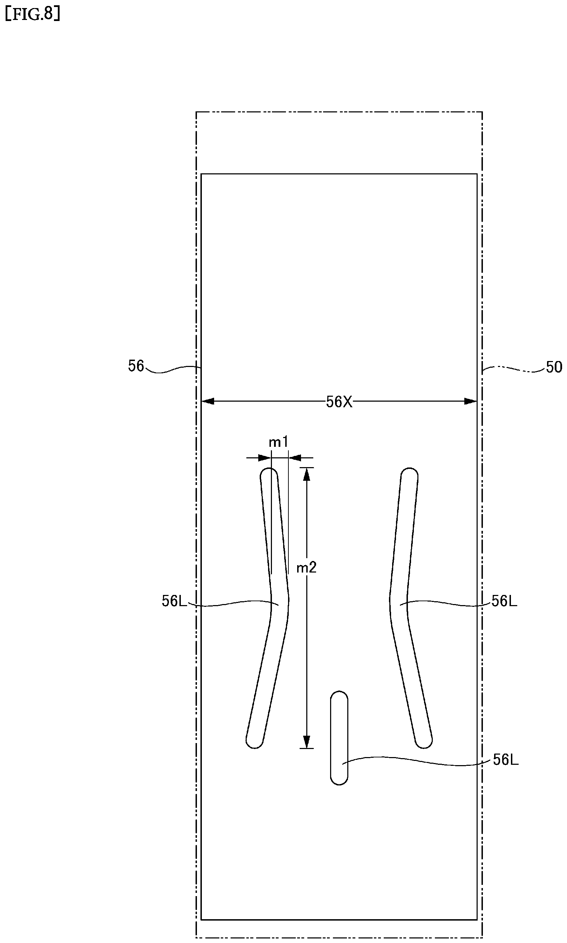

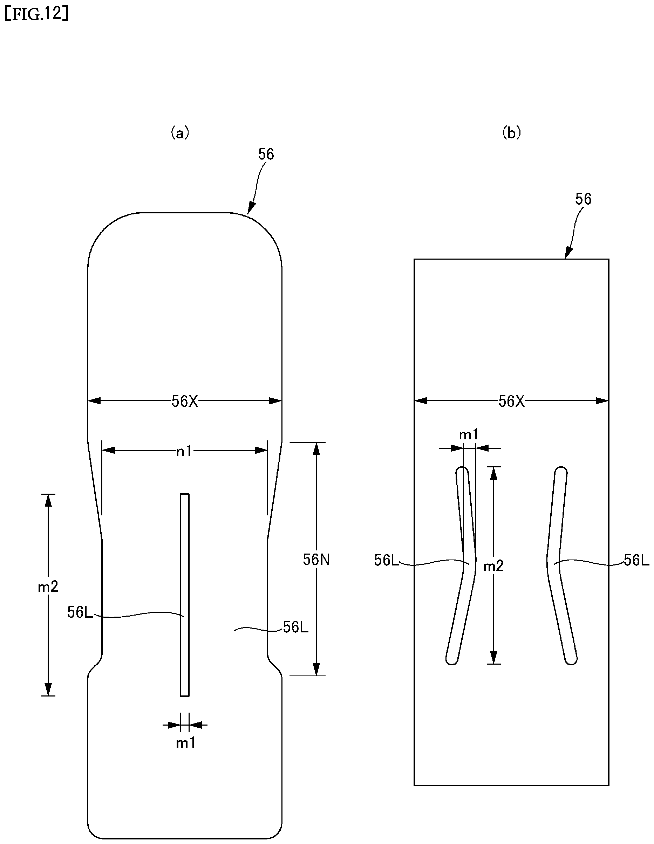

For assisting ensured amount of absorption in the crotch section M, the absorber body 56 may preferably be in a generally rectangular shape as in the embodiment illustrated in Fig. 8. Alternatively, as in the embodiment illustrated in Fig. 12(a), for improved fitting in the crotch section M, the width of the absorber body 56 may be narrowed in the crotch section M compared to the widths forward and backward of the crotch section M to impart a narrowed shape to the absorber body 56. In this case, for assisting ensured amount of absorption in the crotch section M, it is preferred that the narrowest width n1 of the absorber body 56 in the crotch section M is 0.85 times or more of the maximum width 56X of the absorber body 56.

Note that, when the absorber body 56 has a narrowed zone 56n as will be discussed later, the crotch section M refers to the range in the front-back direction LD including this narrowed zone 56n, whereas, when the absorber body 56 does not have the narrowed zone 56n but the contour of the diaper in the spread state has a narrowed zone as in the illustrated embodiment, the crotch section M refers to the range in the front-back direction LD including this narrowed zone of the contour (in the illustrated embodiment, between the front outer member 12F and the back outer member 12B), or where the absorber body 56 has neither of the narrowed zones, the crotch section M refers to the range in the middle in the front-back direction LD corresponding to 20 to 30% the entire length of the product. The extensions forward and backward of the crotch section M are forward and backward zones, respectively.

### <Low-basis-weight area>

The absorber body 56 may have elongate low-basis-weight areas 56L extending in the front-back direction LD on the lateral sides opposed in the width direction WD of the crotch section M or the like, as in the embodiment illustrated in Figs. 1 to 4, or may not have such low-basis-weight areas 56L, though not shown. A low-basis-weight area 56L denotes an area having a low basis weight, and does not encompass an area which is only compressed in the thickness direction TD without any difference in basis weight, like highly-compressed areas 51 as will be discussed later. A low-basis-weight area 56L may be in the form of a slit penetrating the absorber body 56 in the thickness direction TD, or may be in the form of a recess with smaller amounts of accumulated pulp fibers and high-absorbent polymer particles, as in the illustrated embodiment, which is preferred for ensuring amount of absorption. The recess may be formed in the top face or the under face of the absorber body 56. By providing the absorber body 56 with the low-basis-weight areas 56L, flexure of the absorber body 56 along the low-basis-weight areas 56L is promoted to improve fitting of the absorbent element 50 in the crotch section M. It suffices that the total basis weight of the pulp fibers and the high-absorbent polymer particles in the low-basis-weight areas 56L is lower than the total basis weight of the pulp fibers and the high-absorbent polymer particles in the areas other than the low-basis-weight areas 56L, and may be, for example, 0.1 to 0.5 times the total basis weight of the pulp fibers and the high-absorbent polymer particles in the areas other than the low-basis-weight areas 56L.

A low-basis-weight area 56L, as long as being elongate in the front-back direction LD, may extend linearly in the front-back direction LD, or may be curved laterally outwards with increasing proximity to its ends in the front-back direction LD, as in the illustrated embodiment. Further, each of the front and back ends of a low-basis-weight area 56L may be in a suitable shape, for example, may be in the form of a straight line as in the embodiment shown in Fig. 12(a), may be protruded arcuately (semicircular or the like) as in the embodiment shown in Fig. 8, or may have rounded corners with a straight line therebetween, though not shown. The width m1 of a low-basis-weight area 56L may suitably be decided, and may be, for example, 0.04 to 0.1 time the narrowest width n1 of the absorber body 56 in the crotch section M (entire width 56X where the absorber body 56 is rectangular). The width m1 of a low-basis-weight area 56L may be constant or varied along its length direction. The dimension and arrangement in the front-back direction LD of a low-basis-weight area 56L may suitably be decided. For example, the dimension m2 in the front-back direction LD of a low-basis-weight area 56L may be 50 to 120%, more preferably 50 to 80% of the dimension in the front-back direction LD of the crotch section M. Further, a low-basis-weight area 56L may be within the extent of the crotch section M, or may extend beyond the front and/or back ends of the crotch section M.

One low-basis-weight area 56L may be provided on each side in the width direction WD of the crotch section M as well as in the center of the width direction WD as shown in Fig. 8, only one low-basis-weight area 56L may be provided along the center of the crotch section M as shown in Fig. 12(a), or one low-basis-weight area 56L may be provided only on each side in the width direction WD of the crotch section M as shown in Fig. 12(b).

### <Packing Sheet>

The material of the packing sheet 58 may be crepe paper, nonwoven fabric, polyethylene-laminated nonwoven fabric, perforated sheet, or the like. The nonwoven fabric used for the packing sheet 58 is not particularly limited, and may preferably be SMS or SSMMS nonwoven fabric having at least one meltblown layer interposed between a pair of top and under spunbonded layers. The material of the fibers is not particularly limited and may be, for example, polypropylene fibers or polyethylene/polypropylene bicomponent fibers. The basis weight of the packing sheet 58 may suitably be decided, and may be preferably 5 to 40 g/m², particularly 10 to 30 g/m².

The crepe paper used as the packing paper is not particularly limited, and those having a basis weight of 13 to 20 g/m², particularly 14 to 18 g/m² are preferred. The air permeance of the crepe paper to be determined in accordance with JIS P 8117: 2009 "Paper and bord - Determination of air permeance and air resistance (medium range) - Gurley method" is preferably 1 to 15 seconds.

On the other hand, the crepe paper used as the packing sheet 58 usually has a stretch at break in the front-back direction LD of 20 to 35% and a stretch at break in the width direction WD of 4 to 8% (particularly 5 to 7%), determined in accordance with JIS P 8113: 2006.

How to pack the absorber body with the packing sheet 58 may suitably be decided and, in view of readiness of production or protection against leakage of the high-absorbent polymer particles through the front or back end edge of the resulting packed body, preferably, as in the illustrated embodiment, the packing sheet 58 is wrapped cylindrically around the absorber body 56 to surround its top and under faces as well as both side faces, with the front and back edge portions of the packing sheet extending forwardly and backwardly beyond the absorber body 56, and the overlapped areas and the front and back extensions are preferably joined with joining means, such as a hot melt adhesive or material melt-bonding.

### <Highly-Compressed Area>

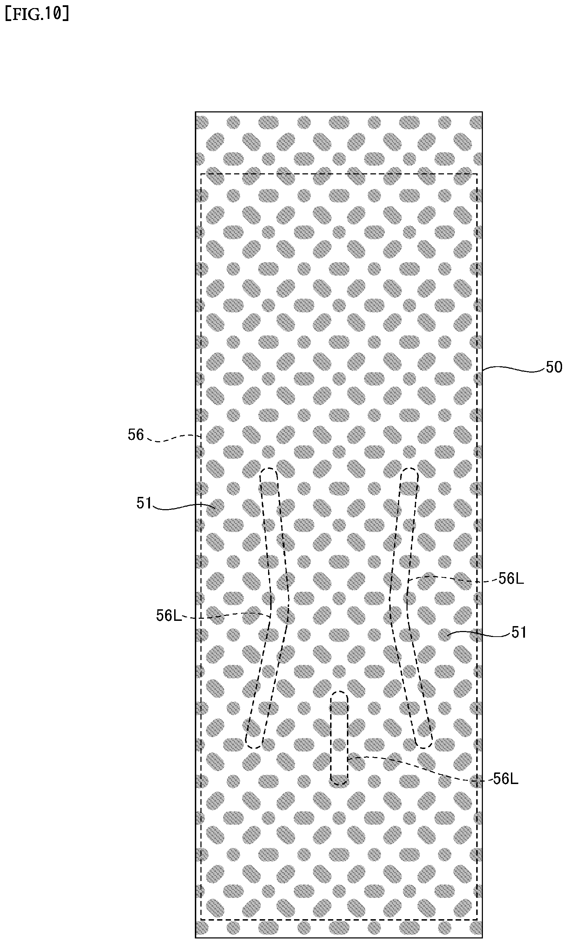

In the absorbent element 50, as shown in Figs. 3, 4, and 9 to 11, highly-compressed areas 51 are arranged at intervals, each compressed in the thickness direction TD from the top face of the absorbent element 50 into the absorber body 56 to form a dent. In the region where the highly-compressed areas 51 are arranged (the minimum bonding rectangle circumscribing all of the highly-compressed areas 51 arranged, surrounded by the sides along the front-back direction LD and along the width direction WD), the areas other than the highly-compressed areas 51 are non-highly-compressed areas 52 with a larger thickness and a lower density compared to those of the highly-compressed areas 51. The highly-compressed areas 51 are areas of a higher density formed through compression by pressurizing (direct pressurizing) into a generally uniform thickness, and constitute the bottoms of dents, whereas the non-highly-compressed areas 52 are areas with a larger thickness and a lower density compared to those of the highly-compressed areas 51. However, even in the non-highly-compressed areas 52, the absorber body is deformed by deformation in the highly-compressed areas dragging the non-highly-compressed areas in the vicinity of the highly-compressed areas to deform, so that the density of a non-highly-compressed area is higher at a location closer to the highly-compressed areas. The non-highly-compressed areas 52 may be partly or entirely compressed in the thickness direction TD at the same time as, before, or after the formation of the highly-compressed areas 51, as long as the non-highly-compressed areas 52 have a larger thickness and a lower density compared to those of the highly-compressed areas 51. The non-highly-compressed areas 52 preferably have no dents in one or both of the top and under faces of the absorbent element 50, but may have dents in one or both of the top and under faces of the absorbent element 50 as long as the non-highly-compressed areas 52 have a larger thickness and a lower density compared to those of the highly-compressed areas 51.

The highly-compressed areas 51 may be provided all over the absorbent element 50 in the front-back direction and the width direction (i.e., the entire surface of the absorbent element 50 is the region where the highly-compressed areas 51 are arranged), or partly in the front-back direction or partly in the width direction of the absorbent element 50. For example, though not shown, the highly-compressed areas 51 may be provided only in the middle zone in the front-back direction LD of the absorbent element 50 including the crotch section M or, in contrast, may be provided only in the zones forward and backward of the middle zone in the front-back direction LD of the absorbent element 50 including the crotch section M, except for the middle zone. Further, the highly-compressed areas 51 in the illustrated embodiment are not dented from the top face of the sheet located on the top side of the absorbent element (in the illustrated embodiment, the top sheet 30 and the intermediate sheet 40) into the absorber body 56, but may be dented from the top face of the absorbent element 50 into the absorber body 56 as a result of compression from the top face of the sheet located on the top side of the absorbent element into the absorber body 56 to form the dents.

When the packing sheet 58 is formed of crepe paper having the stretch at break as discussed above, and a highly-compressed area 51 is excessively small, excessively long in one direction, or has a contour excessively complicated, the crepe paper may be broken at the edges of the highly-compressed areas or between adjacent highly-compressed areas. Further, as being a hardened area, a highly-compressed area 51 which is excessively large may cause insufficient flexibility of the overall absorber body 56, or tough on the skin as if a foreign matter is contained in the absorber body 56 (feeling of a foreign body). Accordingly, it is preferred that each of the highly-compressed area 51 has a contour without any inflection point or folding point. Further, the diameter of the maximum inscribed circle 53 of the contour of any of the highly-compressed areas 51 is preferably 2 to 5 mm, more preferably 2.5 to 3.5 mm. The perimeter of the contour of any of the highly-compressed areas 51 is preferably 1 to 2.5 times, more preferably 1.0 to 2.0 times, particularly preferably 1.0 to 1.6 times the perimeter of the maximum inscribed circle 53 of the contour of the highly-compressed area 51. On the other hand, the diameter of the maximum inscribed circle 54 of the contour of any of the non-highly-compressed areas 52 is preferably 6.5 mm or less, more preferably 6 mm or less, particularly preferably 5 mm or less. The lower limit of the diameter of the maximum inscribed circle 54 of the contour of any of the non-highly-compressed area 52 is preferably 4 mm.

The highly-compressed areas 51 are areas of a higher density formed by compression of the absorbent element 50 in the thickness direction TD through pressurizing (direct pressurizing) into a generally uniform thickness, and constitute the bottoms of dents recessed from the top face or under face of the absorbent element 50. On the other hand, the non-highly-compressed areas 52 are areas with a larger thickness and a lower density compared to those of the highly-compressed areas 51, but even in the non-highly-compressed areas 52, the absorber body is deformed by deformation in the highly-compressed areas dragging the non-highly-compressed areas in the vicinity of the highly-compressed areas to deform, so that the thickness is smaller and the density is higher of a non-highly-compressed area at a location closer to the highly-compressed areas 51. Accordingly, arrangement of the highly-compressed areas 51 of a certain size more densely than in the conventional absorbent element leads to enlargement of the area wherein the thickness of the non-highly-compressed areas 52 is reducing. This causes less strain (applied tensile force is lower) in the packing sheet 58 during formation of the highly-compressed areas 51 therein, resulting in the packing sheet 58 hardly breakable. Further, even in the non-highly-compressed areas 52, the absorber body 56 may advantageously be made thinner compared to the thickness without the highly-compressed areas 51, as a result of deformation in the highly-compressed areas 51 dragging the non-highly-compressed areas 52 in the vicinity of the highly-compressed areas 51 to deform. In view of the above, it is important that each highly-compressed area 51 is shaped to contain a sufficiently large circle. It is also important that each highly-compressed area 51 is not excessively long in one direction, or does not have a contour excessively complicated. It is further important that the diameter of the maximum inscribed circle 54 of the contour of any of the non-highly-compressed areas 52 is small so that the highly-compressed areas 51 are not sparse (i.e., so that the non-highly-compressed areas 52 are not elongated in every direction).

As far as the above-mentioned ranges are met, the area percentage of the highly-compressed areas 51 in the region where the highly-compressed areas 51 are arranged (the sum of the sizes of the highly-compressed areas 51 / the size of the region where the highly-compressed areas 51 are arranged) may suitably be decided and, usually, may be preferably 10 to 35%, more preferably 20 to 35%.

For example, the size of each highly-compressed area 51 may be about 3 to 15 mm². The longer diameter d1 (the length of the long side of the minimum bounding rectangle) of each highly-compressed area 51 may be about 4 to 9 mm, while the shorter diameter d2 (the length of the short side of the minimum bounding rectangle) may be about 2 to 5 mm. Where the four sides of the minimum bounding rectangle of the highly-compressed area 51 are of the same length, the length d3 of the sides may be about 2 to 5 mm. Further, the shortest distance from a highly-compressed area 51 to the closest another highly-compressed area 51 may be about 1.5 to 3.5 mm.

The contour of each highly-compressed area 51 is not particularly limited, and may preferably be in a shape without any inflection point or folding point, such as a circle, an ellipse, or a rounded rectangle (including those having either pair of the opposite sides not being straight but being semicircular), but is not preferably triangular, cloud-shaped, X-shaped, V-shaped, U-shaped, or the like.

Recently, the proportion of high-absorbent polymer particles is often increased for the purpose of thinning the absorber body 56 in size or the like. However, the increased proportion of high-absorbent polymer particles may cause not only deterioration in shape-retaining property of the highly-compressed areas 51, but also difficulties in deformation of the absorber body 56 in the non-highly-compressed areas 52 in the vicinity of the highly-compressed areas 51 by deformation in the highly-compressed areas 51 dragging the non-highly-compressed areas 52 in the vicinity of the highly-compressed areas 51 to deform. Further, the high-absorbent polymer particles swollen by absorbing excrete liquid may tend to adhere to each other firmly to disturb diffusion of the excrete liquid (gel blocking), which may lead to difficulties in improving diffusibility. Thus, with the basis weight of the pulp fibers in the absorber body 56 being 100 to 500 g/m², the ratio by weight of the pulp fibers to the high-absorbent polymer particles in the absorber body 56 may preferably be 45 : 55 to 65 : 35.

The thickness 50t of the absorbent element 50 and the thickness 51t of the highly-compressed areas 51 may suitably be decided, but the back flow protection and the diffusibility may not be improved sufficiently with insufficient compression. Thus, with the basis weight of the pulp fibers and the ratio of the pulp fibers to the high-absorbent polymer particles falling within the above-mentioned ranges, the thickness 50t of the absorbent element 50 (thickness of the non-highly-compressed areas 52) may preferably be 5 to 13 mm. In this case, the thickness 51t of the highly-compressed areas 51 (the minimum thickness where the areas 51 have various thicknesses) may suitably be decided and, usually, may preferably be 30 to 50% of the thickness 50t of the absorbent element 50.

Though not shown, each highly-compressed area 51 may be formed by compressing the absorbent element 50 in the thickness direction TD from is under face into the absorber body 56 to form a dent. In this case, the under face of the absorbent element 50 is provided with the dents. However, as in the illustrated embodiment, with each highly-compressed areas 51 formed by compressing the absorbent element 50 in the thickness direction TD from its top face into the absorber body 56 to form a dent, both the back flow protection and the diffusibility may be improved. That is, as discussed above, the highly-compressed areas 51 are areas of a higher density formed by compression of the absorbent element through pressurizing (direct pressurizing) into a generally uniform thickness, and constitute the bottoms of dents recessed from the top face of the absorbent element 50. On the other hand, the non-highly-compressed areas 52 are areas with a larger thickness and a lower density compared to those of the highly-compressed areas 51, but even in the non-highly-compressed areas 52, the absorber body 56 is deformed by deformation in the highly-compressed areas 51 dragging the non-highly-compressed areas 52 in the vicinity of the highly-compressed areas 51 to deform, so that the density of a non-highly-compressed area 52 is higher at a location closer to the highly-compressed areas 51. Accordingly, capillary action is significant in the highly-compressed areas 51, which results not only in higher liquid retainability compared to that in the vicinity thereof, but also in liquid being sucked toward the highly-compressed areas 51. In this way, arranging the highly-compressed areas 51 more densely than in the conventional absorbent element realizes retention of more excrete liquid more securely and further from the skin, hardly resulting in back flow. Further, the denser arrangement of the highly-compressed areas 51 than in the conventional absorbent element also causes the excrete liquid absorbed in the absorber body 56 to be acted on by the suction through the capillary action in the large number of neighboring highly-compressed areas 51, to diffuse over a wider range.

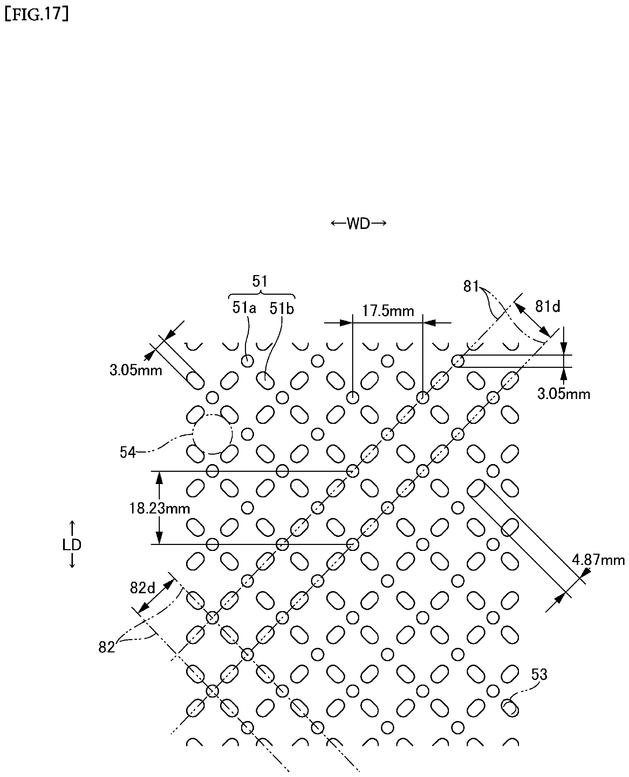

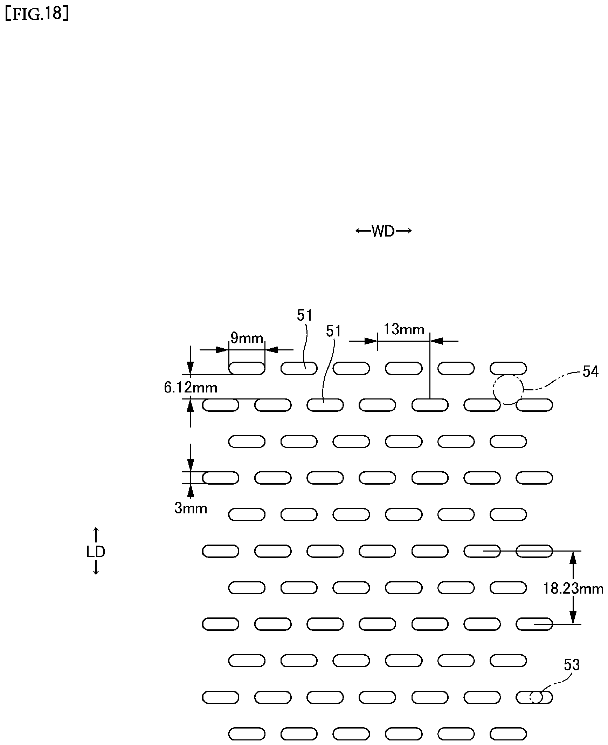

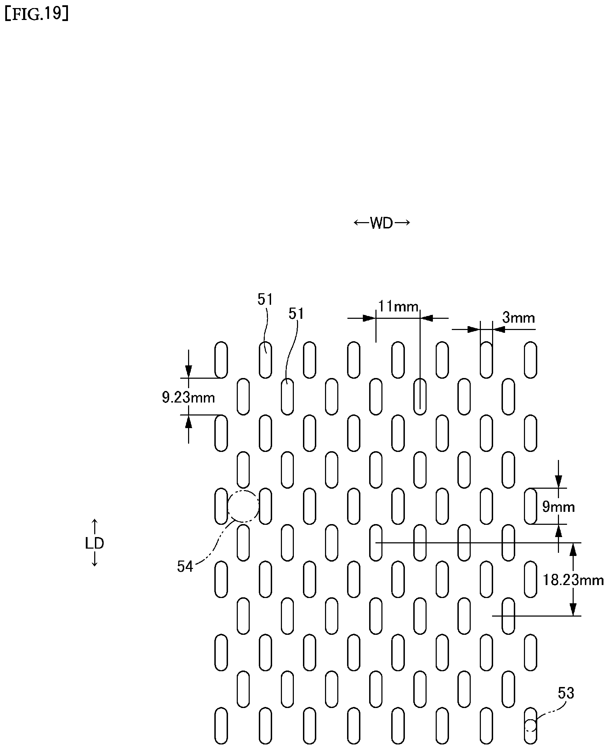

The highly-compressed areas 51 may be arranged in a suitable regular or irregular pattern. For example, the highly-compressed areas 51 may be arranged in a pattern in which the highly-compressed areas 51 provided in dotted lines are arranged in a lattice pattern as shown in Fig. 17, a pattern in which dot-like highly-compressed areas 51 are arranged in a staggered or matrix pattern as shown in Figs. 18 or 19, or the like pattern. Alternatively, though not shown, the highly-compressed areas 51 each extending linearly may be arranged in a lattice pattern.

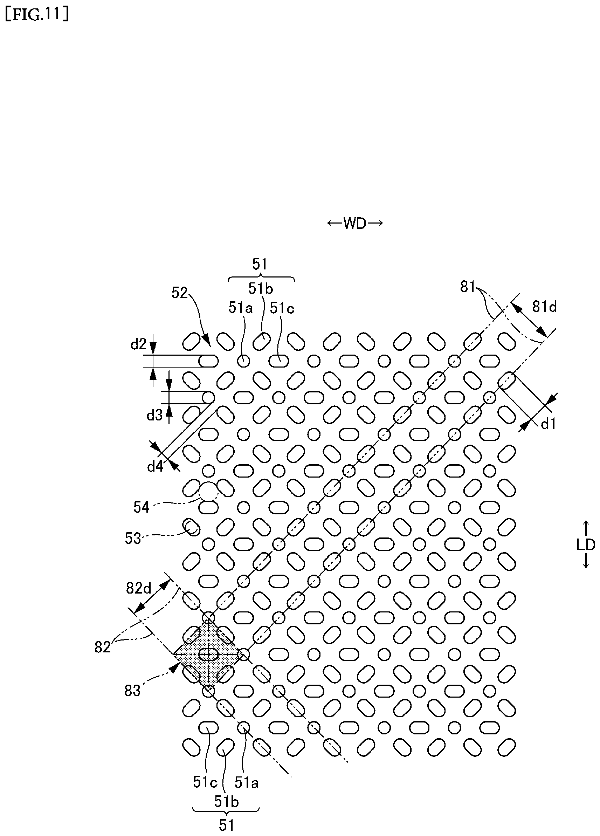

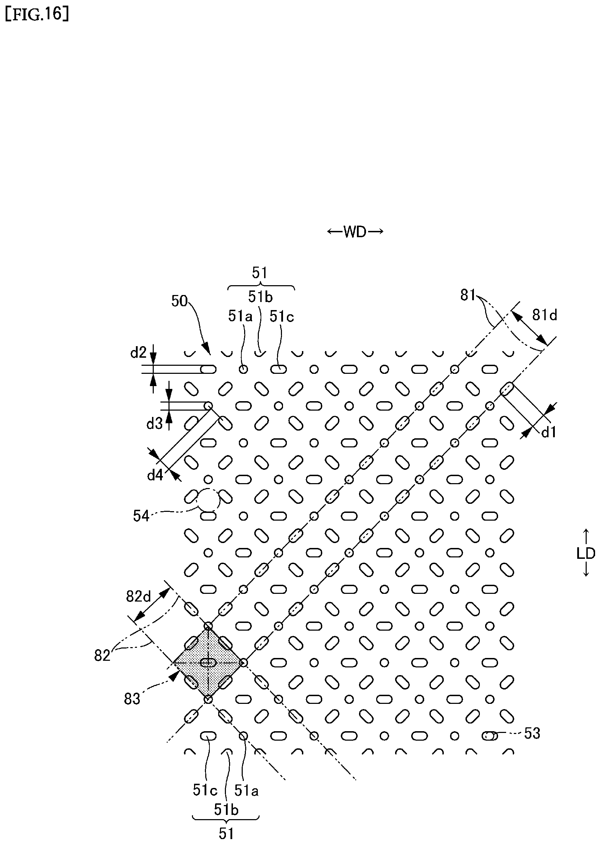

A preferred example of the arrangement pattern of the highly-compressed areas 51 is the embodiment shown in Figs. 11 and 16. In this embodiment, in the region where the highly-compressed areas 51 are arranged, where a virtual lattice is defined in which first virtual lines 81, each extending in a first direction, are arranged at first intervals 81d in a second direction which is inclined at 80 to 90° clockwise with respect to the first virtual lines 81 in plan view, and second virtual lines 82, each extending in the second direction, are arranged at second intervals 82d in the first direction, and minimum virtual rectangles 83 are defined by intersections of the first virtual lines 81 and the second virtual lines 82 as the vertices, the highly-compressed areas 51 are composed of first highly-compressed areas 51a, each located at an intersection of a first virtual line 81 and a second virtual line 82, second highly-compressed areas 51b, each located between two adjacent first highly-compressed areas 51a on a first virtual line 81 or between two adjacent first highly-compressed areas 51a on a second virtual line 82, and third highly-compressed areas 51c, each located at the intersection of the diagonals of a virtual rectangle. Each of the first highly-compressed areas 51a is in the form of a circle having its center at the intersection of a first virtual line 81 and a second virtual line 82, each of the second highly-compressed areas 51b has its center of gravity at the midpoint of a side of a virtual rectangle, and is in the form of a rounded rectangle (or an ellipse) having its long axis extending along the side, and each of the third highly-compressed areas 51c has its center of gravity at an intersection of the diagonals of a virtual rectangle, and is in the form of a rounded rectangle having its long axis extending in the width direction WD (or may be an ellipse, or a circle having its center at an intersection of the diagonals of a virtual rectangle). With this pattern, diffusibility in the direction of the long axis of the second highly-compressed areas 51b (the first and second directions) may be improved, and non-highly-compressed areas 52 linearly continued in the first direction and non-highly-compressed areas 52 linearly continued in the second direction are both provided, so that the absorber body 56 as a whole is easily deformable to the body surface, even if each highly-compressed area 51 is hardened.

When the highly-compressed areas 51 are to be arranged along the virtual lattice of the embodiment shown in Figs. 11 and 16, the dimensions of each highly-compressed area 51 may suitably be decided, and it is preferred that the first highly-compressed areas 51a are 1 to 4 mm in diameter, the second highly-compressed areas 51b are 3 to 6 mm in long axis and are of the same length in short axis as the diameter of the first highly-compressed areas 51a, the third highly-compressed areas 51c have the same dimensions and the same shape as those of the second highly-compressed areas 51b, except that each third highly-compressed area 51c has the long axis aligned to the width direction WD, and the shortest distance d4 between adjacent first and second highly-compressed areas 51a and 51b is 1 to 2 mm, which improves not only the back flow protection and the liquid diffusibility, but also deformability of the absorber body 56. The ratio of the first interval 81d to the second interval 82d is preferably in the range of 0.9 : 1.1 to 1.1 : 0.9, particularly preferably the first and the second intervals 81d and 82d are equal. The first and the second intervals 81d and 82d may be about 10 to 14 mm.

Further, the virtual lattice discussed above is preferably in the form of a rhombic lattice in which the first virtual lines 81 are inclined at 40 to 50° clockwise with respect to the front-back direction LD in plan view, while the second virtual lines 82 are inclined at 40 to 50° counterclockwise with respect to the front-back direction LD in plan view, so that the absorber body 56 is easily deformable when the diaper is worn, and provides excellent liquid diffusibility.

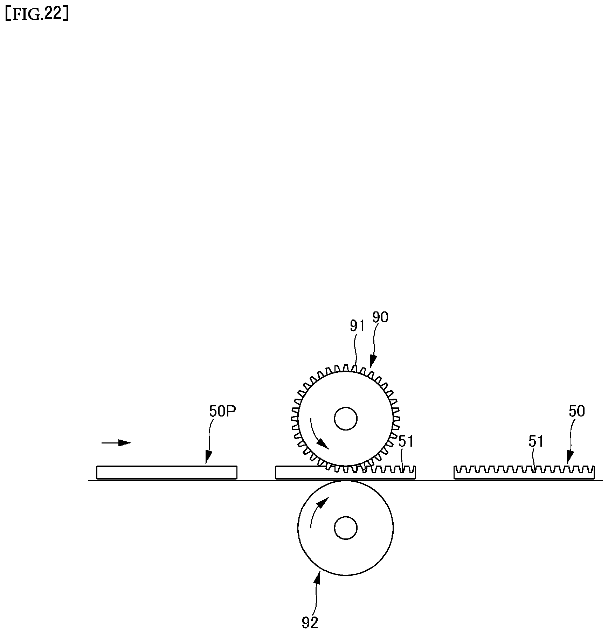

The absorbent element may be manufactured in a commonly known manner. For example, an absorbent element 50 having the highly-compressed areas 51 may be manufactured through the first step of preparing an absorber body 56 by mixing and accumulating pulp fibers and high-absorbent polymer particles, the second step of preparing a packed body 50P by packing the entirety of the absorber body 56 with a packing sheet 58, and the third step of passing the packed body 50P between an anvil roll 90 having on its roll surface a number of protrusions 91 arranged at intervals in a pattern corresponding to the pattern of the highly-compressed areas 51, and a plain roll 92 having a cylindrical face (without protrusions 91) facing the anvil roll 90, as shown in Fig. 22, to compress the areas of the packed body 50P caught between the number of protrusions 91 on the anvil roll 90 and the plain roll 92, to thereby form the highly-compressed areas 51. The highly-compressed areas 51 may be formed under heating of either or both of the anvil roll 90 and the plain roll 92, or without heating. In the third step, only the areas of the packed body 50P that area caught between the number of protrusions 91 on the anvil roll 90 and the plain roll 92 may be compressed, or while the overall packed body 50P is compressed, the areas of the packed body 50P that are caught between the number of protrusions 91 on the anvil roll 90 and the plain roll 92 may be compressed into the deepest.

The dimensions, shape, and arrangement of the tip faces of the protrusions 91 may be similar to the dimensions, shape, and arrangement of the highly-compressed areas 51. The clearance between the anvil roll 90 and the plain roll 92 (minimum gap at a compressing site) may be the same or less than the thickness of the highly-compressed areas 51, e.g., 0.5 to 1.5 m. The pressure in formation of the highly-compressed areas 51 between the anvil roll 90 and the plain roll 92 may suitably be decided, e.g., 0.2 to 0.4 MPa.

The packing sheet 58 is preferably configured such that the machine direction (MD) thereof is along the front-back direction LD for ease of manufacture. In this case, the fiber orientation of the packing sheet 58 is along the front-back direction LD, which remarkably reduces the dry tensile strength in the width direction WD compared to the dry tensile strength in the front-back direction LD. When the dimension in the front-back direction LD of a highly-compressed area 51 is two or more times, particularly three or more times longer than the dimension in the width direction WD thereof (i.e., the shape of the highly-compressed area 51 is elongated in the front-back direction LD), the packing sheet 58 is prone to breakage at both sides in the width direction WD of a highly-compressed area 51. Accordingly, it is preferred that the dimension in the front-back direction LD of each highly-compressed area 51 is less than two times, particularly 1.5 or less times the dimension in the width direction WD thereof, for the packing sheet 58 to be hardly broken.

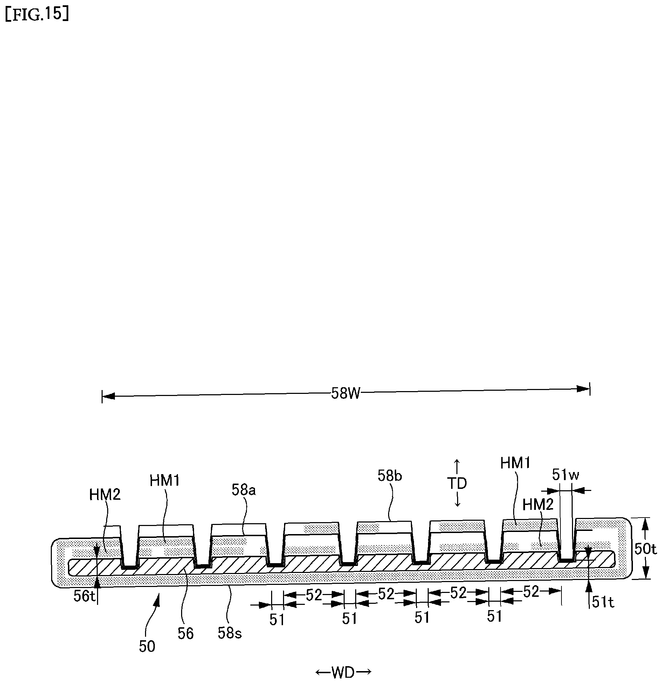

Further, as shown in Fig. 15, the packing sheet 58 has a under section 58s located on the under side of the absorber body 56, a first top section 58a extending continuous from the under section 58s and around a side edge to the underside of the absorber body 56, and a second under section 58b extending continuous from the under section 58s and around the other side edge to the underside of the absorber body 56, the first top section 58a and the second top section 58b are overlapped with each other in a layered section 58W, wherein all of the highly-compressed areas 51 are located in the layered section 58W. In this way, the crepe paper is doubled on the face of the absorber body having the highly-compressed areas 51, so that the crepe paper is given enhanced strength and is hard to be broken upon forming the highly-compressed areas 51, which is preferable.

The shape-retainability of the absorbent element 50 imparted by the highly-compressed areas 51 depends on the retainability of the highly-compressed areas 51 per se, which in turn depends on the joint strength between the packing sheet 58 and the absorber body 56 in the highly-compressed areas 51 and the shape-retainability of the absorber body 56 per se. When adhesion between the packing sheet 58 and the absorber body 56 is achieved using hot melt adhesives HM1 and HM2, while formation of the highly-compressed areas 51 results in increased surface area, a larger amount of hot melt adhesives HM1 and HM2 than usual is required for sufficiently maintaining the joint strength between the packing sheet 58 and the absorber body 56 and the shape-retainability of the absorber body 56 per se. Accordingly, it is preferred that the top face of the absorber body 56 and the interior face of the packing sheet 58 are bonded with 5.0 to 20.0 g/m², particularly 7.5 to 15.0 g/m² of hot melt adhesives MH1 and HM2 at least all over the region where the highly-compressed areas are arranged.

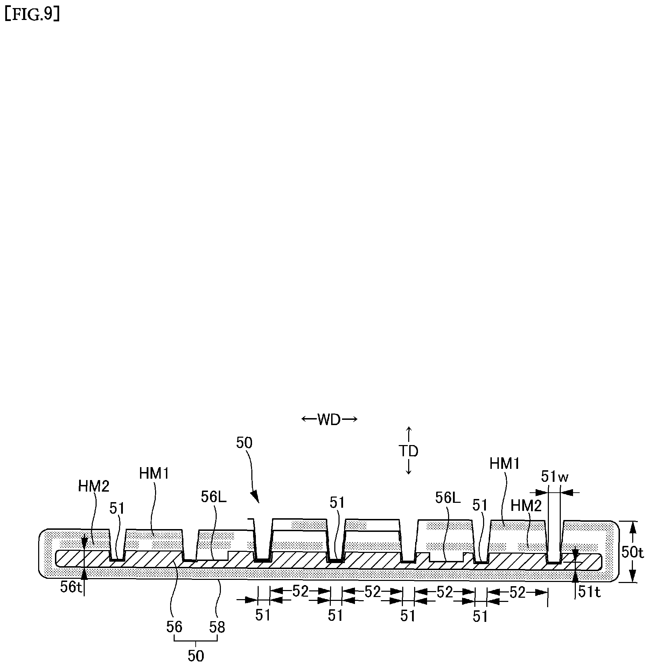

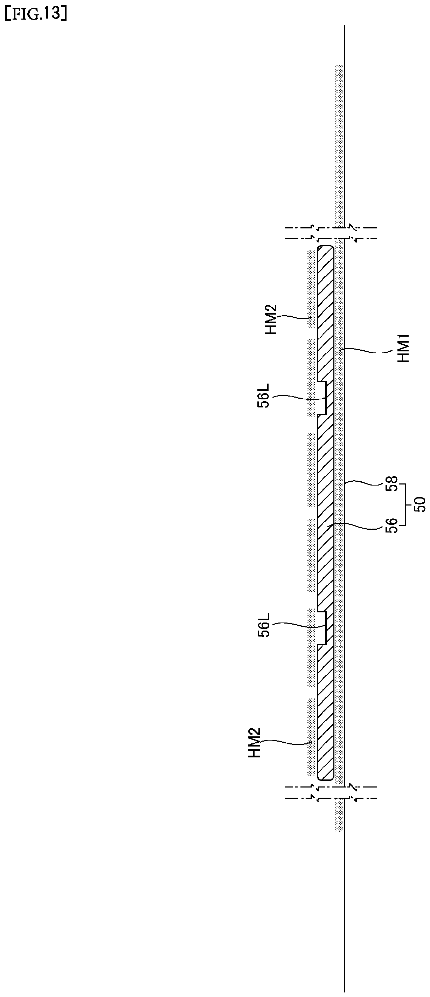

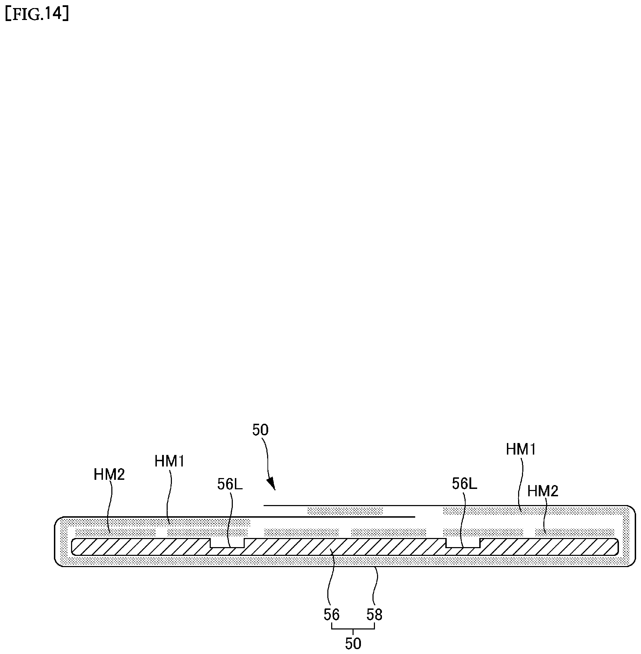

For example, as shown in Fig. 9, when dents of the highly-compressed areas 51 are to be formed in the top face of the absorber body 56, the above-mentioned amount of the hot melt adhesive HM may be achieved by providing two layers of the hot melt adhesives HM1 and HM2 on the underside of the absorber body 56 all over the region where the highly-compressed areas 51 are arramged. Such a structure may be produced by, as shown in Fig. 13, applying a first hot melt adhesive HM1 almost all over the inner face of the packing sheet 58 in the spread state, which is to face the absorber body 56, and then placing the absorber body 56 in the middle in the width direction WD of the first hot melt adhesive HM1 on the packing sheet 58 to adhere the under face of the absorber body 56 to the packing sheet 58 with the first hot melt adhesive HM1. Next, after a second hot melt adhesive HM2 is applied almost all over the top face of the absorber body 56, as shown in Fig. 14, each of the sections of the packing sheet 58 that extend laterally beyond the opposed edges of the absorber body 56 is folded onto the top face of the absorber body 56 to adhere the top face of the absorber body 56 and the folded sections of the packing sheet 58 with the first hot melt adhesive HM1 and the second hot melt adhesive HM2, while the overlapped sections of the packing sheet 58 are adhered together with the first hot melt adhesive HM1, and then as shown in Fig. 9, the highly-compressed areas 51 arranged in a lattice pattern are formed by embossing, to thereby produce the structure.

The absorbent element 50 may be free of any other sheet layers, such as paper or nonwoven fabric, between the packing sheet 58 and the absorber body 56 (naturally, the adhesive layers may be present) as shown in Fig. 9 for cost reduction, or may have other sheet layers on at least one of the top and under faces of the absorber body 56.

### <Standup Gather Parts>

Each of the standup gather parts 60 has a standup zone 68 which stands up from the inner member 200 on a lateral side thereof, and is brought into contact with the wearer from his/her groin along the round-leg profile up to the buttocks to avoid side leakage. The standup gather parts 60 may be omitted as required. The standup gather parts 60 in the illustrated embodiment each has a root-side portion 60B obliquely standing up toward the center in the width direction, and a distal-side portion 60A than the intermediate portion obliquely standing up outwards in the width direction. However, the standup gather parts are not limited to this, and suitable modification may be made, so that, for example, each of the gather part as a whole may stand up toward the center in the width direction.

More specifically, each of the standup gather parts 60 in the illustrated embodiment is composed of a strip-like gathered sheet 62 which is of the length equal to the front-back dimension of the inner member 200, and is folded back in two in the longitudinal direction to form a distal edge along the folded line, and a plurality of elongate gathering elastic members 63 held between the layers in the folded area and the vicinity thereof and arranged at intervals in the width direction WD, with each of the gathering elastic members 63 being in its stretched state in the longitudinal direction. The proximal portion located opposite from the distal portion of each standup gather part 60 (the edge portion opposite in the width direction WD from the folded-edge portion of the sheet) is referred to as a root portion 65, which is fixed to a lateral side of the inner member 200, and the portion other than the root portion 65 is referred to as a main body portion 66 (the portion on the folded-edge-portion side) extending from the root portion 65. The main body portion 66 has the root-side portion 60B extending toward the center in the width direction, and a distal-side portion 60A folded along the distal edge of the root-side portion 60B and extending outwards in the width direction. Front and back end zones of each main body portion 66 are fixed in a laid-down state onto the top sheet 30 on one of its opposed sides to form laid-down zones 67, while the middle zone in the front-back direction between these zones is a non-fixed standup zone 68, wherein at least the distal portion thereof contains gathering elastic members 63 in a stretched state, each extending along the front-back direction LD.

The standup zone 68 of each of the standup gather parts 60 configured in this way, stands up to abut the skin as shown by the arrow in Fig. 3 by means of the contraction force of the gathering elastic members 63. In particular, with the root portions 65 positioned on the underside of the inner member 200, the standup zones 68 stand up to open outwards in the width direction in and near the crotch section, so that the standup gather parts 60 are brought into surface contact with around the legs to improve fitting. Alternatively, the root portions 65 may be fixed on the top side of the inner member 200, i.e., on the top face of the top sheet 30 on its lateral sides.

As with the standup gather parts 60 of the illustrated embodiment, when the main body portion 66 has a bent structure composed of the root-side portion 60B extending toward the center in the width direction and a distal-side portion 60A folded along the distal edge of the root-side portion 60B and extending outwards in the width direction, the distal-side portion 60A and the root-side portion 60B are joined together in the laid-down state in the laid-down zones 67, while the root-side portion 60B is joined in the laid-down state to the top sheet 30. For the joining between the facing surfaces in the laid-down zones 67, at least one of a hot melt adhesive and means of material melt-bonding, such as heat sealing and ultrasonic sealing, may be employed. In this case, the joining between the root-side portion 60B and the top sheet 30 and the joining between the distal-side portion 60A and the root-side portion 60B may be established by same or different means. For example, it is preferred to establish the joining between the root-side portion 60B and the top sheet 30 with a hot melt adhesive, and the joining between the distal-side portion 60A and the root-side portion 60B by material melt-bonding.

The gathered sheets 62 may preferably be made of nonwoven fabric having flexibility and superior in uniformity and concealability, such as spunbonded nonwoven fabric (SS, SSS, or the like), SMS nonwoven fabric (SMS, SSMMS, or the like), or meltblown nonwoven fabric, optionally subjected to water-repellent treatment with silicone or the like, as necessary. In this case, the fiber basis weight of the nonwoven fabric may preferably be about 10 to 30 g/m². Further, though not shown, a waterproof film may be interposed between the layers of the double-folded gathered sheet 62.

The gathering elastic members 63 may be rubber threads. When Spandex rubber threads are to be used, the fineness thereof may be preferably 470 to 1240 dtex, more preferably 620 to 940 dtex. The stretch rate of the gathering elastic members 63 in the fixed state may be preferably 150 to 350%, more preferably 200 to 300%. The number of the gathering elastic members 63 may be preferably two to six, more preferably three to five. The interval of the gathering elastic members 63 may appropriately be 3 to 10 mm. In this way, the region containing the gathering elastic members 63 is easily brought into surface contact with the skin. The gathering elastic members 63 may be arranged not only in the distal side, but also in the root side.

In the standup zone 68 of the standup gather part 60, at least one of a hot melt adhesive in various application manners and fixing means of material melt-bonding, such as heat sealing and ultrasonic sealing, may be employed for adhering the inner and outer layers of the gathered sheet 62 together or fixing the gathering elastic members 63 therebetween. If the inner and outer layers of the gathered sheet 62 are bonded all over, flexibility of the sheet is impaired, so that it is preferred not to bond or to bond weakly the areas of the inner and outer layers other than the bonding areas of the gathering elastic members 63. In the illustrated embodiment, by applying a hot melt adhesive only to the circumferential surfaces of the gathering elastic members 63 using application means, such as comb guns or surewrap nozzles, and holding the gathering elastic members 63 between the inner and outer layers of the gathered sheet 62, not only the fixing of the gathering elastic members 63 to the inner and outer layers of the gathered sheet 62, but also the fixing between the inner and outer layers of the gathered sheet 62, may be achieved by means of only the hot melt adhesive applied on the circumferential surfaces of the gathering elastic members 63.

Similarly, for fixing the laid-down zones 67, at least one of a hot melt adhesive in various application manners and means of material melt-bonding, such as heat sealing and ultrasonic sealing, may be employed.

### <Side Flap>

As shown in Figs. 1 to 4, the inner member 200 is provided, on its opposed lateral sides, width side flaps 70 extending laterally outwards of the absorber body 56, and each side flap 70 preferably has a side stretchable zone SG stretchable in the front-back direction. Each side flap 70 in the illustrated embodiment has one or a plurality of elongate side elastic members 73 arranged at intervals, each extending in the front-back direction LD, a first sheet layer 71 facing the external faces of the side elastic members 73, and a second sheet layer 72 facing the internal faces of the side elastic members 73.

The first sheet layer 71 and the second sheet layer 72 may be made of any sheet material without particular limitation, and suitable nonwoven fabric, such as those usable for the standup gather parts 60 or the outer members 12F, 12B, may be selected. In the illustrated embodiment, the gathered sheet 62 of each standup gather parts 60 is extended to form the first sheet layer 71 and the second sheet layer 72. In this case, the front and back ends of the side flaps 70 are coincident with the front and back ends of the standup gather parts 60 (i.e., in this case, the front and back ends of the inner member 200), as will be discussed later.

The side elastic members 73 are not particularly limited, and elongate elastic members similar to the gathering elastic members 63 may be used. The stretch rate of the side elastic members 73 in the fixed state may be preferably 150 to 350%, more preferably 200 to 270%. The number of the side elastic members may be preferably two to sixteen, more preferably six to ten. The interval of the side elastic members may appropriately be 5 to 10 mm.

The side elastic members 73 are fixed to the first sheet layer 71 and the second sheet layer 72. For adhering the first sheet layer 71 and the second sheet layer 72 together or fixing the side elastic members 73 therebetween, a hot melt adhesive HM in various application manners or fixing means of material melt-bonding, such as heat sealing and ultrasonic sealing, may be employed. If the joined area between the first sheet layer 71 and the second sheet layer 72 is too large, flexibility of the sheets is impaired, so that it is preferred not to bond or to bond weakly the areas of the first and second sheet layers other than the bonding areas of the side elastic members 73. In the illustrated embodiment, by applying hot melt adhesive HM only to the circumferential surfaces of the side elastic members 73 using application means, such as comb guns or surewrap nozzles, and holding the side elastic members 73 between the first and the second sheet layers 71 and 72, not only the fixing of the side elastic members 73 to the first and the second sheet layers 71 and 72, but also the fixing between the first and the second sheet layers 71 and 72, may be achieved by means of only the hot melt adhesive HM applied on the circumferential surfaces of the side elastic members 73.

In the illustrated embodiment, the sheet material of the first sheet layer 71 and the sheet material of the second sheet layer 72 are folded along the side edge of the side flap 70, and the folded part is fixed on the under face of the liquid impervious sheet 11 (formed into a sealed bag). This fixing may be achieved with a hot melt adhesive HM as in the illustrated embodiment, or by material melt-bonding.

The side flaps may be omitted.

### <Test 1 for Confirming Effects>

Samples of the absorbent element were fabricated under the various conditions as shown in Table 1, and tested for breakage of packing sheet, dispersing behavior, back flow under unpressurized conditions, back flow under pressure, and absorption rate. Note that the basis weight of the packing sheet, dry tensile strength of the packing sheet, and the stretch at break of the packing sheet shown in Tables 1 and 3 refer to the values of the material before the compressed areas were formed.

Various dimensions of Sample 1 were as follows:
Length d3 of first highly-compressed area 51a: 3 mm
Longer diameter d1 of second and third highly-compressed areas: 4.82 mm
Shorter diameter d2 of second and third highly-compressed areas: 3 mm
First interval 81d: 12.63 mm
Second interval 82d: 12.63 mm
Angle of intersection between first and second virtual lines 81 and 82: 90 degrees

Various dimensions of Sample 1 were as follows:
Length d3 of first highly-compressed area 51a: 2 mm
Longer diameter d1 of second and third highly-compressed areas: 3.82 mm
Shorter diameter d2 of second and third highly-compressed areas: 2 mm
First interval 81d: 12.63 mm
Second interval 82d: 12.63 mm
Angle of intersection between first and second virtual lines 81 and 82: 90 degrees

In Samples 1 to 7, the highly-compressed areas were formed all over the top face of the absorbent element under 0.4 MPa. Sample 8 was a comparative example without the highly-compressed areas. Specifications other than those shown in Table 1 were the same for every sample.

### <Evaluation of Packing Sheet Breakage>

Surface of a sample of the absorbent element was visually observed, and evaluated as X when breakage was observed almost all over the absorbent element surface at similar locations of the highly-compressed areas, evaluated as Δ when small breakage was observed in part of the absorbent element surface, but no breakage was observed in most of the surface, and evaluated as O when no breakage was observed at all.

### <Test for Confirming Diffusion Behavior>

This test was conducted according to the following procedure:
(1) A sample was placed on a horizontally-placed flat plate with the top face (the face having the dents of highly-compressed areas) of the sample up, and fixed to the plate with adhesive tapes applied at the four corners of the sample in the spread state.
(2) About 5 g of Brilliant Blue FCF (food dye, Blue No. 1) manufactured by Kiriya Chemical Co., Ltd. (packed in powder form) was dissolved in 300 ml of artificial urine to prepare a blue test liquid.
(3) A syringe (acrylic cylinder of 23 mm in inner diameter, 25 mm in outer diameter, and 100 mm in height) was placed on the top face of a sample in its center (center both in the longitudinal and the width directions) and held vertically, and 1.5 cm³ of the blue test liquid was poured at a time through the upper end opening of the syringe using a measuring cylinder.
(4) After the lapse of 5 minutes, the syringe was removed, still images were taken of the top face and the under face of the sample, the image data thus taken were read using an image analysis software, and the size of the blue area on each of the top and under faces of the sample was determined.

### <Absorption Rate Test>

This test was conducted according to the following procedure:
(1) A sample was placed on a horizontally-placed flat plate with the top face (the face having the dents of highly-compressed areas) of the sample up, and fixed to the plate with adhesive tapes applied at the four corners of the sample in the spread state.
(2) A syringe (acrylic cylinder of 3 mm in inner diameter, 6 mm in outer diameter, and 220 mm in height) was held vertically above the center (center both in the longitudinal and the width directions) of the top face of a sample, with the distance between the lower end of the syringe and the top face of the sample being 10 mm.
(3) Through the upper end opening of the syringe, 70 cm³ of the blue test liquid was poured at 7 cm³/sec using a measuring cylinder, and the absorption time (in second) required from the start of the pouring until the blue test liquid was gone (completely absorbed) from the top face of the sample was measured with a stopwatch, which was taken as the first absorption rate. Whether the liquid was completely absorbed or not was decided through visual observation by the operator.
(4) After the sample was left to stand for 30 minutes, the operation in (3) was conducted again, and the second absorption rate was determined.

### <Back Flow Test under Unpressurized Conditions>

This test was conducted according to the following procedure:
(1) A sample was placed on a horizontally-placed flat plate with the top face (the face having the dents of highly-compressed areas) of the sample up, and fixed to the plate with adhesive tapes applied at the four corners of the sample in the spread state.
(2) A syringe (acrylic cylinder of 23 mm in inner diameter, 25 mm in outer diameter, and 100 mm in height) was placed on the top face of a sample in its center (center both in the longitudinal and the width directions) and held vertically.
(3) Through the upper end opening of the syringe, 50 cm³ of the blue test liquid was poured at 7 cm³/sec using a measuring cylinder, and the sample was left as it was from the start of the pouring until the blue test liquid was gone (completely absorbed) from the top face of the sample.
(4) Ten sheets of Quantitative Filter Paper No. 2 (square of 100 mm long and 100 mm wide) manufactured by ADANTEC TOYO KAISHA, LTD. were weighed (in grams) with an electronic balance to three places of decimals.
(5) After the lapse of 30 minutes from the complete absorption of the blue test liquid poured in (3) above, the ten filter papers were aligned and placed in layers on the site where the blue test liquid was poured, and a 1 kg weight was placed on top and left to stand. Here, the weight had a flat bottom face in a square shape of 100 mm long and 100 mm wide. The filter papers and the weight were placed on the top face of the sample such that the longitudinal and lateral directions of the filter papers and the weight are aligned to the front-back direction and the width direction, respectively, of the sample, and that the centers of the filter papers and the weight were placed in the center of the top face of the sample.
(6) After the lapse of 1 minute from the placement of the weight in (5) above, all of the filter papers were removed, and weighed (in grams) to two places of decimals, and the value of the weight before absorption measured in (4) was subtracted from the value of the weight after absorption to take the amount of back flow under unpressurized conditions.

### <Back Flow Test under Pressure>

This test was conducted according to the following procedure:
(1) A sample was placed on a horizontally-placed flat plate with the top face (the face having the dents of highly-compressed areas) of the sample up, and fixed to the plate with adhesive tapes applied at the four corners of the sample in the spread state.
(2) A syringe (acrylic cylinder of 23 mm in inner diameter, 25 mm in outer diameter, and 100 mm in height) was placed on the top face of a sample in its center (center both in the longitudinal and the width directions) and held vertically.
(3) Through the upper end opening of the syringe, 50 cm³ of the blue test liquid was poured at 7 cm³/sec using a measuring cylinder, the the sample was left as it was from the start of the pouring until blue test liquid was gone (completely absorbed) from the top face of the sample, and after the lapse of 30 minutes, another 50 cm³ of the blue test liquid was poured at 7 cm³/sec through the upper end opening of the syringe using the measuring cylinder.
(4) After the lapse of 20 minutes from the complete absorption of the blue test liquid of the second pouring, a 3 kg weight was placed on the site where the blue test liquid was poured, and left to stand for 10 minutes. Here, the weight had a flat bottom face in a square shape of 100 mm long and 100 mm wide, and was placed on the top face of the sample such that the longitudinal and lateral directions of the weight are aligned to the front-back direction and the width direction, respectively, of the sample, and that the center of the weight was placed in the center of the top face of the sample.
(5) Thirty sheets of Quantitative Filter Paper No. 2 (square of 100 mm long and 100 mm wide) manufactured by ADANTEC TOYO KAISHA, LTD. were weighed (in grams) with an electronic balance to three places of decimals.
(6) After the step in (4), the thirty sheets of filter papers, centered and aligned, were held between the weight and the top face of the sample and, after the lapse of 20 seconds, all of the filter papers were removed and weighed (in grams) to two places of decimals, and the value of the weight before absorption measured in (5) was subtracted from this measured value of the weight after absorption to take the amount of back flow under pressure.

**Table 1**

| Sample No. | | 1 | 2 | 3 | 4 | 5 | 6 | 7 | 8 |
|---|---|---|---|---|---|---|---|---|---|
| Basis weight of absorber body | g/m² | 500 | ← | ← | ← | ← | ← | ← | ← |
| Pulp content in absorber body | wt% | 60 | ← | ← | ← | ← | ← | ← | ← |
| SAP content in absorber body | wt% | 40 | ← | ← | ← | ← | ← | ← | ← |
| Material of packing sheet | | Crepe paper | ← | ← | ← | ← | ← | ← | ← |
| Basis weight of packing sheet | g/m² | 18 | ← | ← | ← | ← | ← | ← | ← |
| Dry tensile strength of packing sheet | Front-back direction N/m | 9.6 | ← | ← | ← | ← | ← | ← | ← |
| | Width direction N/m | 3.6 | ← | ← | ← | ← | ← | ← | ← |
| Stretch at break of packing sheet | Front-back direction % | 26 | ← | ← | ← | ← | ← | ← | ← |
| | Width direction % | 6.0 | ← | ← | ← | ← | ← | ← | ← |
| Pattern of compressed areas | | Fig. 11 | Fig. 16 | Fig. 17 | Fig. 18 | Fig. 19 | Fig. 20 | Fig. 21 | Blank |
| Shape of compressed area | | Circle, rounded rectangle | ← | ← | Rounded rectangle | ← | X-shaped | See Fig. 21 | - |
| Size of one compressed area | mm2 | Circle: 7.07 | Circle: 3.14 | Circle: 7.07 | 25.07 | ← | 168.00 | Bent: 175 | - |
| | | Rounded rectangle: 12.5 | Rounded rectangle: 6.78 | Rounded rectangle: 12.9 | | | | Linear (front-back): 118 | |
| | | | | | | | | Linear (oblique): 38.8 | |
| Area percentage | % | 27.9 | 14.8 | 21.1 | 20.9 | 25.4 | 24.2 | 17.7 | - |
| Diameter of maximum inscribed circle 54 of non-compressed area 52 | mm | 4.9 | 5.9 | 9.9 | 7.5 | 8.0 | 15.2 | 27.0 | - |
| Diameter of maximum inscribed circle 53 of compressed area 51 | mm | 3.0 | 2.0 | 3.1 | 3.0 | 3.0 | 5.1 | 3.2 | - |
| Circumference of compressed area 51 | mm | Circle: 9.42 | Circle: 6.28 | Circle: 9.42 | 21.4 | 21.4 | 113 | Bent: 122 | - |
| | | Rounded rectangle: 13.1 | Rounded rectangle: 9.92 | Rounded rectangle: 13.2 | | | | Linear (front-back): 83.4 | |
| | | | | | | | | Linear (oblique): 30.6 | |
| Complexity of shape of compressed area | Times | 1.0-1.4 | 1.0-1.6 | 1.0-1.4 | 2.3 | 2.3 | 7.1 | 3.0-12.0 | - |
| Aspect ratio of compressed area front-back direction / width direction | | 0.6-1.0 | 0.5-1.0 | 1.0 | 0.3 | 3.0 | 1.0 | 1.0-3.5 | - |

The results of the tests are shown in Table 2.

**Table 2**

| Sample No. | | 1 | 2 | 3 | 4 | 5 | 6 | 7 | 8 |
|---|---|---|---|---|---|---|---|---|---|
| Dispersed area (top face): A | cm2 | 20.8 | 18.4 | 17.5 | 22.9 | 18.9 | 21.2 | 23.7 | 15.1 |
| Dispersed area (under face): B | cm² | 19.7 | 16.3 | 20.7 | 23.8 | 17.0 | 25.5 | 30.8 | 13.7 |
| Back flow under unpressurized conditions (30 min) | 9 | 0.029 | 0.032 | 0.055 | 0.062 | 0.059 | 0.059 | 0.056 | 0.055 |
| Back flow under pressure | 9 | 3.9 | 4.6 | 5.5 | 5.6 | 5.6 | 5.8 | 6.6 | 6.5 |
| Absorption rate (1st) | see | 19.7 | 20.1 | 21.6 | 20.5 | 20.7 | 20.0 | 18.5 | 19.4 |
| Absorption rate (2nd) | see | 20.1 | 20.2 | 22.4 | 20.0 | 20.2 | 21.0 | 21.3 | 20.7 |
| Evaluation of breakage | | O | △ | X | △ | X | X | X | |
| Thickness of absorbent element | mm | 3.8 | 4.2 | 4.4 | 4.3 | 4.3 | 4.3 | 4.3 | 4.8 |

Samples 1 and 2 were not only superior in diffusibility (dispersed into a sufficiently large area) similar to Samples 3 to 7 and compared to Sample 8, but also lower in the amounts of back flow under unpressurized conditions and of back flow under pressure, compared to Samples 3 to 8. Further, Samples 1 and 2 were superior in absorption rate similar to Samples 3 to 6 and compared to Samples 7 and 8. Samples 1 and 2 were also superior in protection against crepe paper breakage.

### <Test 2 for Confirming Effect>

Samples of the underpants-type disposable diapers as shown in Figs 1 to 6 were fabricated using the various absorbent elements as shown in Table 3, and tested for back flow under unpressurized conditions, back flow under pressure, and absorption rate as discussed above. Note that the various dimensions and arrangements of the highly-compressed areas of Sampe 9 were the same as in Sample 1. Samples 10 and 11 were comparative examples without the highly-compressed areas. Specifications other than those shown in Table 3 were the same for every sample.

**Table 3**

| Sample No. | | 9 | 10 | 11 |
|---|---|---|---|---|
| Basis weight of absorber body | g/m² | 396 | 400 | 330 |
| Pulp content in absorber body | wt% | 49 | 48 | 42 |
| SAP content in absorber body | wt% | 51 | 52 | 58 |
| Material of packing sheet | | Crepe paper | ← | ← |
| Basis weight of packing sheet | g/m² | 18 | ← | ← |
| Dry tensile strength of packing sheet | Front-back direction N/m | 9.6 | ← | ← |
| | Width direction N/m | 3.6 | ← | ← |
| Stretch at break of packing sheet | Front-back direction % | 26 | ← | ← |
| | Width direction % | 6.0 | ← | ← |
| Pattern of compressed areas | | Fig. 11 | - | - |
| Shape of compressed area | | Circle, rounded rectangle | - | - |
| Size of one compressed area | mm2 | Circle: 7.07 | | |
| | | Rounded rectangle: 12.5 | - | - |
| Area percentage | % | 27.9 | - | - |
| Diameter of maximum inscribed circle 54 of non-compressed area 52 | mm | 4.9 | - | - |
| Diameter of maximum inscribed circle 53 of compressed area 51 | mm | 3.0 | - | - |
| Circumference of compressed area 51 | mm | Circle: 9.42 | - | - |
| | | Rounded rectangle: 13.1 | | |
| Complexity of shape of compressed area | Times | 1.0-1.4 | - | - |
| Aspect ratio of compressed area front-back direction / width direction | | 0.6-1.0 | - | - |

The results of the tests are shown in Table 4.

**Table 4**

| Sample No. | | 9 | 10 | 11 |
|---|---|---|---|---|
| Back flow under unpressurized conditions (30 min) | 9 | 0.07 | 0.07 | 0.07 |
| Back flow under pressure | 9 | 4.40 | 6.01 | 7.32 |
| Absorption rate (1st) | sec | 16 | 17 | 17 |
| Absorption rate (2nd) | sec | 18 | 21 | 20 |

The present tests on diapers as the samples showed the trend similar to Test 1 for Confirming Effect on the absorbent elements only. That is, Sample 9 is lower in amounts of back flow under unpressurized conditions and back flow under pressure, and superior in absorption rate, compared to Samples 10 and 11.

### <Explanation of Terms in the Specification>

The following terms appearing in the present specification shall have the following means unless otherwise specified herein.

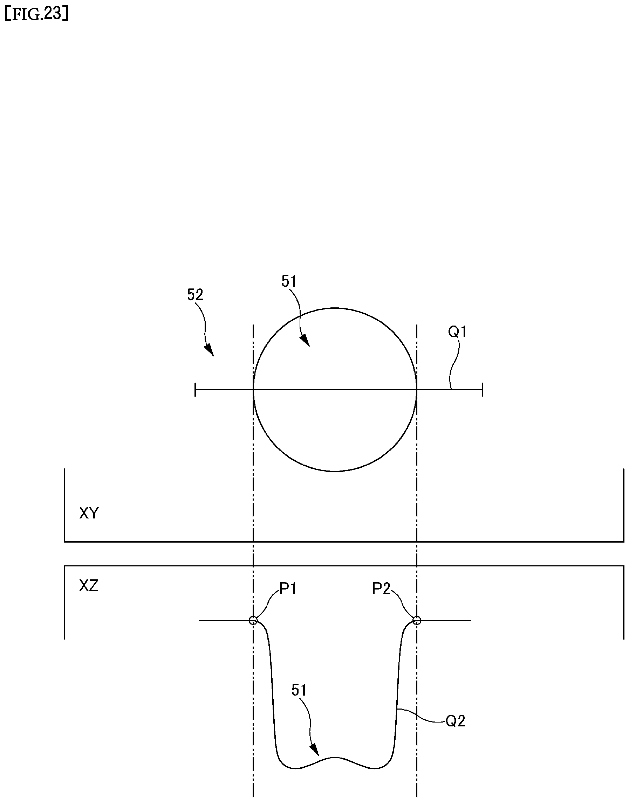

- The "front-back direction" refers to the direction shown by the reference sign LD (longitudinal direction) in the figures, whereas the "width direction" refers to the direction shown by the reference sign WD (transverse direction) in the figures, and the front-back direction and the width direction are orthogonal to each other.
- The "top side" refers to the side, when the article is worn, closer to the skin of the wearer, whereas the "underside" refers to the side, when the article is worn, away from the skin of the wearer.
- The "top face" refers to the face, when the article is worn, closer to the skin of the wearer, whereas the "under face" refers to the face, when the article is worn, away from the skin of the wearer.
- The "stretch rate" refers to a value with respect to the natural length being 100%. For example, a 200% stretch rate is synonymous with stretch in two folds.
- The "artificial urine" is a mixture of 2 wt% urea, 0.8 wt% sodium chloride, 0.03 wt% calcium chloride dihydrate, 0.08 wt% magnesium sulfate heptahydrate, and 97.09 wt% ion-exchanged water.
- The "gel strength" is determined as follows. To 49.0 g of artificial urine, 1.0 g of high-absorbent polymer is added and stirred with a stirrer. The resulting gel is left in a chamber with constant temperature and humidity at 40 °C at 60% RH for 3 hours, and then the temperature is returned to the ordinary temperature. The gel strength is measured in a curd meter (Curdmeter-MAX ME-500 manufactured by I. techno Engineering).
- The "basis weight" is determined as follows. A specimen or test piece is preliminarily dried, left in a laboratory or in apparatus under the standard conditions (23 ± 1 °C temperature and 50 ± 2% relative humidity in the testing location) until constant mass is attained. The preliminary drying refers to attaining constant mass from a specimen or test piece in the environment at a temperature of 100 °C. No preliminary drying may be performed on fibers with an official regain of 0.0%. From the test piece of the constant mass, a specimen of 100 mm × 100 mm size is cut out using a sampling template (100 mm × 100 mm). The weight of the specimen is measured and multiplied by 100 times to calculate the weight per 1 m², which is taken as the basis weight.
- The "thickness" of a thick member, such as the absorber body 56 and the absorbent element 50, is measured using a thickness meter manufactured by OZAKI MFG. CO., LTD. (PEACOCK, dial thickness gauge Model H (measurement range of 0 to 10 mm, circular terminal with measurement area of 10 mm in diameter, measuring force of about 1.7 N, pressure of about 21.7 KPa)), with the specimen and the thickness meter kept horizontally. The thickness of the absorbent element 50 (non-highly-compressed areas 52) is measured as follows. That is, ten of the non-highly-compressed areas 52 of the absorbent element 50 are selected, in each of which the inscribed circle of the contour of a non-highly-compressed area 52 is maximum, and the thickness measured with the center of the head of the thickness gauge registered with the center of the inscribed circle of each of the ten areas is taken as the thickness of the absorbent element 50 (non-highly-compressed areas 52) .
- The "thickness" of the highly-compressed areas 51 is determined by subtracting the depth of the highly-compressed areas 51 from the thickness of the absorbent element 50 (non-highly-compressed areas 52).
- The "depth" of the highly-compressed areas 51 is measured using Wide-Area 3D Measurement Microscope VR-3200 manufactured by KEYENCE CORPORATION or an equivalent thereof and an image analysis software VR-H2A manufactured by KEYENCE CORPORATION or an equivalent thereof. In principle, the measurements are made at a magnification of 12 folds in the field of view of 24 mm by 18 mm, provided that the magnification and the area of the field of view may be adjusted depending on the size of the highly-compressed areas 51. Specific manner of measurement is explained with reference to Fig. 23. Using the above-mentioned software and the like, the depth profile (total profile) of one highly-compressed area 51 in the image area shown in planar view (area X in the figure) is obtained along line Q1 passing the center (or the center of gravity, where the shape of the object is not circular) of the highly-compressed area 51. From the primary profile of this embossing depth profile, the surface roughness components of the wavelength shorter than λc: 800 µm (provided that λc stands for "a filter defining the boundary of the roughness component and the waviness component" provided in 3.1.1.2 in JIS-B0601) are filtered out with a low-pass filter to obtain Profile Q2 indicated in the image area shown in cross-sectional view (area Y in the figure). On this profile Q2, two dent edge points P1, P2 where the profile Q2 is almost flat, or convexed upwards and curved most sharply, are identified, and the minimum value of the height between these dent edge points P1, P2 is determined. On the other hand, the average of the height values at the dent edges P1, P2 is taken as the maximum value of the height. In this way, by subtracting the minimum value of the height from the maximum value of the height, the depth of the highly-compressed area 51 is obtained. These two dent edge points P1, P2 are visually selected. In selecting these points, the image in the planar view of the highly-compressed area 51 being measured, may be referred to. The measurement thus discussed above is made of ten arbitrarily-selected highly-compressed areas 51 in the top face, and the average thereof is eventually taken as the result.
- The "thickness" of a thin sheet, such as nonwoven fabric, is automatically measured using an automatic thickness meter (KES-G5 handy compression tester program) under a load of 0.098 N/cm² with the compression area of 2 cm².
- The "area percentage" refers to the percentage of the area of the targets per unit area, and is obtained and expressed in percentage by dividing the sum of the areas of the targets (e.g., the highly-compressed areas 51) in the entire objective region (e.g., the under face of the absorbent element 50) by the area of that objective region. The dimensions, the area, and the like of the highly-compressed areas 51 are determined by taking still images of the face of the absorbent element 50 having the highly-compressed areas 51, and reading the image data thus taken using an image analyzer software. Here, the objective part of the highly-compressed area 51 for measurement is automatically selected based on shading, or visually selected, if unable to be selected automatically.
- The water absorption is determined in accordance with JIS K7223 - 1996 "Testing method for water absorption capacity of super absorbent polymers".
- The water absorption rate is defined as the "time spent until the end point is reached" in carrying out JIS K7224 - 1996 "Testing method for water absorption rate of super absorbent polymers" using 2 g of superabsorbent polymer and 50 g of saline.
- The "spread state" refers to the state in which an article is spread flatly without contraction (including any contraction, such as contraction by means of elastic members) or slack.
- The size or dimensions of each part refers to the size or dimensions not in the natural length state but in the spread state, unless otherwise specified.
- A test or measurement shall be, in the absence of description about environmental conditions, performed in a laboratory or in apparatus under the standard conditions (23 ± 1 °C temperature and 50 ± 2% relative humidity in the testing location).

### INDUSTRIAL APPLICABILITY

The present invention is applicable to disposable diapers, such as underpants-type disposable diapers, tape-type disposable diapers, and pad-type disposable diapers, as well as absorbent articles in general having the crotch section (preferably, also having extensions either one or both of forward and backward of the crotch section), such as sanitary napkins.

### DESCRIPTION OF REFERENCE SIGNS

11: liquid-impervious sheet
12A: side seal
12B: back outer member
12E: waist extension
12F, 12B: outer member
12F: front outer member
12S, 12H: sheet material
13: covering nonwoven layer
16, 19: under-waist elastic member
17: waist elastic member
18: unnecessary elastic member
20: inner member joint area
200: inner member
30: top sheet
40: intermediate sheet
50: absorbent element
50P: packed body
51: highly-compressed area
51a: first highly-compressed area
51b: second highly-compressed area
51c: third highly-compressed area
52: non-highly-compressed area
56: absorber body
56L: low-basis-weight area
58: packing sheet
60: standup gather part
60A: distal-side portion
60B: root-side portion
62: gathered sheet
63: gathering elastic member
67: laid-down zone
68: standup zone
70: side flap
71: first sheet layer
72: second sheet layer
73: side elastic member
81: first virtual line
82: second virtual line
90: anvil roll
91: protrusion
92: plain roll
A1: non-stretchable region
A2: stretchable region
B: back body section
C: buttocks-covering zone C
F: front body section
HM: hot melt adhesive
HM1: first hot melt adhesive
HM2: second hot melt adhesive
L: intermediate region
LD: front-back direction
LO: leg opening
M: crotch section
SG: side stretchable zone
T: lower torso region
TD: thickness direction
U: under-waist zone
W: waist zone
WD: width direction
WO: waist opening

## Claims

1. An absorbent article having a crotch section, the absorbent article comprising:
an absorbent element comprising an absorber body arranged in a range in a front-back direction including the crotch section, and a packing sheet of crepe paper packing the absorber body,
wherein the absorber body has been formed by mixing and accumulating pulp fibers and high-absorbent polymer particles,
wherein the absorbent element has highly-compressed areas arranged at intervals, each having been formed by compressing the absorbent element in a thickness direction from its top face or under face into the absorber body to form a dent,
wherein, in a region where the highly-compressed areas are arranged, areas other than the highly-compressed areas are non-highly-compressed areas with a larger thickness and a lower density compared to those of the highly-compressed areas,
wherein each of the highly-compressed areas has a contour without any inflection point or folding point, a diameter of a maximum inscribed circle of a contour of any of the highly-compressed areas is 2 to 5 mm, and a perimeter of a contour of any of the highly-compressed areas is 1 to 2.5 times a perimeter of the maximum inscribed circle of the contour of the highly-compressed area, and
wherein a diameter of a maximum inscribed circle of a contour of any of the non-highly-compressed areas is 6.5 mm or less.

2. The absorbent article according to claim 1,
wherein a basis weight of the pulp fibers in the absorber body is 100 to 500 g/m2,
wherein a ratio by weight of the pulp fibers to the high-absorbent polymer particles in the absorber body is 45 : 55 to 65 : 35,
wherein the packing sheet is crepe paper having a stretch at break in a front-back direction of 20 to 35% and a stretch at break in the width direction of 4 to 8%, as determined in accordance with JIS P 8113: 2006,
wherein a thickness of the non-highly-compressed areas is 5 to 15 mm, and
wherein a thickness of the highly-compressed areas is 30 to 50% of the thickness of the non-highly-compressed areas.

3. The absorbent article according to claim 1 or 2,
wherein the highly-compressed areas are composed only of highly-compressed areas formed by compressing the absorbent element in a thickness direction from its top face into the absorber body to form dents.

4. The absorbent article according to claim 1 or 2,
wherein, in a region where the highly-compressed areas are arranged, where a virtual lattice is defined in which first virtual lines, each extending in a first direction, are arranged at first intervals in a second direction which is inclined at 80 to 90° clockwise with respect to the first virtual lines in plan view, and second virtual lines, each extending in the second direction, are arranged at second intervals in the first direction, and
minimum virtual rectangles are defined by intersections of the first virtual lines and the second virtual lines as vertices,
the highly-compressed areas are composed of first highly-compressed areas, each located at an intersection of a first virtual line and a second virtual line, second highly-compressed areas, each located between adjacent first highly-compressed areas on a first virtual line or between adjacent first highly-compressed areas on a second virtual line, and third highly-compressed areas, each located at an intersection of diagonals of a virtual rectangle,
wherein each of the first highly-compressed areas is in a form of a circle having its center at an intersection of a first virtual line and a second virtual line,
wherein each of the second highly-compressed areas has its center of gravity at a midpoint of a side of a virtual rectangle, and is in a form of an ellipse or a rounded rectangle having its long axis extending along the side, and
wherein each of the third highly-compressed areas has its center of gravity at the intersection of the diagonals of a virtual rectangle, and is in a form of an ellipse or a rounded rectangle having its long axis extending in the width direction, or a circle having its center at the intersection of the diagonals of a virtual rectangle.

5. The absorbent article according to claim 4,
wherein the first highly-compressed areas are 1 to 4 mm in diameter,
wherein the second highly-compressed areas are 3 to 6 mm in long axis, and are of same length in short axis as the diameter of the first highly-compressed areas,
wherein the third highly-compressed areas have same dimensions and same shape as those of the second highly-compressed areas, except that each third highly-compressed area has a long axis aligned to the width direction, and
wherein a shortest distance between adjacent first and second highly-compressed areas is 1 to 2 mm.

6. The absorbent article according to claim 5,
wherein the virtual lattice is a rhombic lattice in which the first virtual lines are inclined at 40 to 50° clockwise with respect to the front-back direction in plan view, while the second virtual lines are inclined at 40 to 50° counterclockwise with respect to the front-back direction in plan view.

7. A method for producing an absorbent element having an absorber body formed by mixing and accumulating pulp fibers and high-absorbent polymer particles, and a packing sheet of crepe paper packing the absorber body, the method comprising:
a first step of preparing an absorber body by mixing and accumulating the pulp fibers and the high-absorbent polymer particles,
a second step of preparing a packed body by packing an entirety of the absorber body with the packing sheet, and
a third step of holding the packed body between an anvil roll having on its roll surface protrusions arranged at intervals and a plain roll having a cylindrical face facing the anvil roll, and compressing areas of the packed body caught between the number of protrusions on the anvil roll and the plain roll from the top face or the under face of the packed body into the absorber body to form dents,
wherein, when an arrangement of tip faces of the protrusions of the anvil roll developed in a plan view, in a region where the tip faces are arranged, a diameter of a maximum inscribed circle of a contour of the tip face of any of the protrusions is 2 to 5 mm, a perimeter of a contour of the tip face of any of the protrusions is 1 to 2.5 times a perimeter of the maximum inscribed circle of the contour of the tip face of the protrusion, and a diameter of a maximum inscribed circle of a contour of any area in the region, where the tip faces are arranged, other than the tip faces is 6.5 mm or less.
